# EUROPÄISCHE PATENTANMELDUNG

(11) **EP 1 801 152 A1**
(43) Veröffentlichungstag der Anmeldung: **27.06.2007**
(21) Anmeldenummer: 05090349.1
(22) Anmeldetag: 23.12.2005
(51) Int. Cl.: C08L 3/02

(54) **Reisstärken mit verbesserten Qualitätseigenschaften**

(71) Anmelder: Bayer CropScience GmbH, 65929 Frankfurt/Main (DE)
(72) Erfinder: Frohberg, Claus, 14532 Kleinmachnow (DE); Schmidt, Ralf-Christian, 14469 Potsdam (DE)
(74) Vertreter: Kossmann, Jochen

(57) **Zusammenfassung**

Die vorliegende Erfindung betrifft Reisstärke und Reismehl mit verbesserten Qualitätseigenschaften, Reiskörner, die diese Reisstärke enthalten sowie Reispflanzen, die diese Reiskörner tragen.

## Beschreibung

Die vorliegende Erfindung betrifft Reisstärke und Reismehl mit verbesserten Qualitätseigenschaften, Reiskörner, die diese Reisstärke enthalten sowie Reispflanzen, die diese Reiskörner tragen.

Reis bildet das wichtigste Nahrungsmittel für mehr als die Hälfte der Erdbevölkerung. In einzelnen Ländern Asiens beträgt der Reisanteil an der gesamten Nahrungsaufnahme etwa 80%. Die Jahresproduktion weltweit liegt bei 550 Millionen Tonnen Reis.
Das Reiskorn besteht zu etwa 76% aus Stärke und zu etwa 7-8% aus Eiweiß. Es enthält nur 1,3% Fett und zahlreiche Spurenelemente (0,6%), wie Phosphor, Eisen und Magnesium.

Die Reisart mit der wirtschaftlich größten Bedeutung ist *Oryza sativa,* deren Grundsorten sich in zwei Gruppen aufteilen:
die *"indica"*-Gruppe, sie beinhaltet nur Langkornreis und
die "*japonica*"-Gruppe, sie beinhaltet Mittel- und Rundkornreis.

Der Langkornreis (auch "Brühreis", trocken kochende Reissorten) kommt primär aus Indien oder Java; der Rundkornreis (wie "Milchreis", d.h. klebrig kochende Reissorten) primär aus Japan. Dazwischen liegen die Sorten aus China und Südostasien.
Bei allen Sorten unterscheidet man wiederum zwei Hauptarten: durchscheinendes Korn oder trübes Korn. Diese unterscheiden sich in der Zusammensetzung ihrer Stärke: die Stärke des durchscheinenden Reises besteht zu etwa 20% aus Amylose und zu 80% aus Amylopektin, die des trüben Reises dagegen fast nur aus Amylopektin.

Die Koch- und Verzehreigenschaften sind zum größten Teil durch den Amylosegehalt des Reis-Endosperms bestimmt. Sorten mit niedrigem Amyloseanteil sind nach dem Kochen feucht und klebrig während Körner mit hohem Amylosegehalt beim Kochen trocken und flockig werden (H. ten Have in Hoffmann Mudra Plarre (HMP) 1985: Lehrbuch der Züchtung landw. Kulturpflanzen, Band 2, S. 110-123).

Die Kornqualität spielt für den Verbraucher, aber auch für die Mühlenindustrie eine große Rolle; Korneigenschaften wie Korngröße, -form und -beschaffenheit sind wichtige Merkmale, da sie den Ertrag an gemahlenem Reis und seinen Bruchanteil beeinflussen (ten Have 1985, supra).

Reismehl ist relativ geschmacksneutral und von daher als Grundlage für Produkte mit mildem Geschmack und auch als Beimischung gut geeignet. Aufgrund seiner hypoallergenen Wirkung lässt es sich auch gut für Baby- bzw. Allergikernahrung verwenden.

Neben Ölen, Fetten und Proteinen stellen Polysaccharide die wesentlichen nachwachsenden Rohstoffe aus Pflanzen dar. Eine zentrale Stellung bei den Polysacchariden nimmt neben Cellulose die Stärke ein, die einer der wichtigsten Speicherstoffe in Höheren Pflanzen ist.

Das Polysaccharid Stärke ist ein Polymer aus chemisch einheitlichen Grundbausteinen, den Glucosemolekülen. Es handelt sich dabei jedoch um ein sehr komplexes Gemisch aus unterschiedlichen Molekülformen, die sich hinsichtlich ihres Polymerisationsgrades, des Auftretens von Verzweigungen der Glucoseketten und deren Kettenlängen unterscheiden, die darüber hinaus derivatisiert, z.B. phosphoryliert sein können. Daher stellt Stärke keinen einheitlichen Rohstoff dar. Man unterscheidet insbesondere die Amylose-Stärke, ein im wesentlichen unverzweigtes Polymer aus α-1,4-glycosidisch verknüpften Glucosemolekülen, von der Amylopektin-Stärke, die ihrerseits ein komplexes Gemisch aus unterschiedlich verzweigten Glucoseketten darstellt. Die Verzweigungen kommen dabei durch das Auftreten von zusätzlichen α-1,6-glycosidischen Verknüpfungen zustande.

Die funktionellen Eigenschaften der Stärke werden neben dem Amylose/Amylopektin-Verhältnis und dem Phosphatgehalt stark beeinflusst durch das Molekulargewicht, das Muster der Seitenkettenverteilung, den Gehalt an Ionen, den Lipid- und Proteingehalt, die mittlere Stärkekorngröße sowie die Stärkekorn-Morphologie etc. Als wichtige funktionelle Eigenschaften sind hierbei beispielsweise zu nennen die Löslichkeit, das Retrogradationsverhalten, das Wasserbindevermögen, die Filmbildungseigenschaften, die Viskosität, die Verkleisterungseigenschaften, die Gefrier/Tau-Stabilität, die Säurestabilität, die Gelfestigkeit etc..

Die grundlegenden Synthesewege, die zum Aufbau von Stärke führen, sind lediglich im Groben bekannt. Es gibt jedoch eine Reihe von Teilschritten, bei denen die exakten Mechanismen, die zum Aufbau der Stärkekörner und zur Synthese der Stärke führen, bislang nicht geklärt und somit noch Gegenstand der Forschung sind.

Die Stärkesynthese in pflanzlichen Zellen findet in den Plastiden statt. In photosynthetisch aktiven Geweben sind dies die Chloroplasten, in photosynthetisch inaktiven, stärkespeichernden Geweben die Amyloplasten. Wichtige an der Stärkesynthese beteiligte Enzyme sind die R1-Proteine (=alpha-Glucan-Wasser-Dikinase, E.C. 2.7.9.4; Lorberth et al. (1998) Nature Biotechnology 16: 473-477), Stärkesynthasen sowie die Verzweigungsenzyme (= "Branching enzymes" = BE; siehe z.B. Ponstein et al., Plant Physiol. 29 (1990), 234-241). Verzweigungsenzyme katalysieren die Einführung von α-1,6-Verzweigungen in lineare α-1,4-Glucane. Bei den Stärkesynthasen sind verschiedene Isoformen beschrieben, die alle eine Polymerisierungsreaktion durch Übertragung eines Glucosylrestes von ADP-Glucose auf α-1,4-Glucane katalysieren.

Eine Übersicht zu nativen Stärken, isoliert aus verschiedenen Pflanzenspezies, die eine Veränderung von an der Stärkebiosynthese beteiligten Enzymen aufweisen, findet sich bei Kossmann und Lloyd (2000, Critical Reviews in Plant Sciences 19(3): 171- 226).

Aus WO 03/023024 sind Reisstärken bekannt, die eine DSC T-onset Temperatur von bis zu 69,5°C bzw. eine DSC T-peak Temperatur von bis zu 73,6°C aufweisen, diesbezüglich wurden insgesamt ca. 400 Reis-Varietäten der Gruppen *japonica* und *indica* analysiert.

Umemoto et al. (2002, Theor. Appl. Genet. 104:1-8) beschreiben die Analyse von rückgekreuzten Inzuchtlinien (back cross inbred lines) zwischen einer *japonica -* Varietät (Nipponbare) und einer *indica*-Varietät (Kasalath). Aus ihren Ergebnissen folgern sie, dass es sich bei dem alk(t), gel(t) bzw. acl(t) Locus, der für die unterschiedlichen Gelatinisierungstiefsttemperaturen (DSC T-onset) zwischen *japonica* und *indica* Varietäten verantwortlich ist, um die Stärkesynthase Isoform SSIIa handeln könnte.

In WO 03/023024 wird eine Reis Transformante (#78-1) der Varietät *japonica* (Kinmaze) beschrieben, in welche ein Gen der Stärkesynthase IIa (SSIIa) aus der indica-Form IR36 transformiert worden ist. Die daraus resultierenden Veränderungen des Amylopektin Seitenkettenprofils werden dargestellt und zeigen als Folge eine Angleichung des *japonica* Profils an das der *indica*-Varietät (Abb.22 in WO 03/023024).

Weder Phosphatgehalte, Amylosegehalte noch rheologische Eigenschaften der Reisstärken oder Mehle werden in der WO 03/023024 beschrieben.

In einer kürzlich publizierten Veröffentlichung (Nakamura et al., (2005) PMB; 58(2): 213-27) wird erneut der Zusammenhang zwischen der durch die SSIIa verursachte Veränderung des Amylopektin-Seitenkettenprofils und der DSC T-onset-Temperatur der Stärken anhand der Werte für die Transformanten und entsprechenden "Empfänger"- und "Spender"-Linien dargestellt (Abb. 6B). In Abb. 6B werden die Temperaturen für DSC T-onset der Stärken der erzeugten SSIIa-Transformanten dargestellt. In keinem Fall wird ein DSC T-onset von mehr als 70°C erreicht. Der höchste dort angegebene T-onset liegt bei 69,5°C, wie auch in WO 03/023024 (S. 21-24) beschrieben.

Damit lehren weder WO 03/023024 noch Nakamura et al. (2005) wie Reisstärken erzeugt werden können, deren DSC T-onset Temperatur über 70°C hinausgehen.

Eine höhere DSC T-onset Temperatur ist jedoch erwünscht, da sie ein wichtiges Merkmal für eine verbesserte thermische Stabilität von Stärken und damit auch für die durch Hitzeeinwirkung bedingte Veränderung der kristallinen Struktur darstellt.

Daher war Aufgabe der vorliegenden Erfindung, Reisstärken und Reismehle mit neuen Eigenschaften, insbesondere einer verbesserten thermischen Stabilität, sowie Mittel und Verfahren zu ihrer Herstellung, zur Verfügung zu stellen.

Diese Aufgabe wird durch die Bereitstellung der in den Patentansprüchen bezeichneten Ausführungsformen gelöst.

Gegenstand der vorliegenden Erfindung ist eine Reisstärke, die eine DSC T-onset Temperatur zwischen 70°C und 80°C aufweist.

Die Wärmeeigenschaften der Stärke des Reis-Endosperms sowie der Reismehle lassen sich durch das Wärmefluss-Kalorimetrieverfahren (Differential Scanning Calorimetry = DSC) analysieren. Diese werden als Gelatinisierungstemperatur mit den Werten für die DSC T-onset (=Gelatinisierungstiefsttemperatur) sowie für DSC T-peak (=Gelatinisierungshöchsttemperatur) dargestellt.

Unter dem Begriff "DSC T-onset Temperatur" ist daher im Zusammenhang mit der vorliegenden Erfindung diejenige Temperatur zu verstehen, welche den Beginn der Phasenumwandlung der Stärke- bzw. der Mehlprobe darstellt. Sie wird charakterisiert als der Schnittpunkt zwischen der Verlängerung der Basislinie und der an die ansteigende Flanke des Peaks angelegten Tangente durch den Wendepunkt.

Überraschenderweise synthetisieren die erfindungsgemäßen Reispflanzen Stärken mit DSC T-onset Temperaturen, die zwischen 70°C und 80°C liegen. Die erfindungsgemäße Reisstärke weist insbesondere ein DSC T-onset zwischen 72 und 79°C auf, bevorzugt zwischen 74°C und 79°C, ganz besonders bevorzugt zwischen 76°C und 78°C.

In einer weiteren Ausführungsform weisen die erfindungsgemäßen Reisstärken eine erhöhte DSC T-peak Temperatur (DSC T-peak) auf.

Überraschenderweise synthetisieren die erfindungsgemäßen Reispflanzen Stärken mit einer DSC T-peak Temperatur, die zwischen 80 und 87°C, bevorzugt zwischen 81 und 86°C, liegt. Besonders bevorzugt ist eine erfindungsgemäße Reisstärke, deren DSC T-peak Temperatur zwischen 82°C und 83°C beträgt.

Unter dem Begriff "DSC T-peak Temperatur" wird im Zusammenhang mit der vorliegenden Erfindung die Temperatur bezeichnet, bei der die DSC-Kurve ein Maximum erreicht hat und die erste Ableitung der Kurve Null ist.

Die Bestimmung der "DSC T-onset"- sowie der "DSC T-peak" - Temperatur erfolgt im Zusammenhang mit der vorliegenden Erfindung nach der unten beschriebenen Methode ("Thermische Analyse von Reismehl/-stärke mittels Wärmefluß-Kalorimetrieverfahren").

Es war für den Fachmann äußerst überraschend, dass die DSC-T-onset-Temperatur der erfindungsgemäßen Reisstärke derart stark erhöht war. Insbesondere deshalb, da die erfindungsgemäßen Reisstärken gleichzeitig einen erhöhten Stärkephosphatgehalt aufweisen. Denn bislang wurde postuliert, dass eine Erhöhung des Phosphorylierungsgrades zu einer Destabilisierung der Doppelhelices sowie der kristallinen Ordnung im Stärkekorn führt, welches eine Reduktion der DSC T-onset Temperatur zur Folge haben sollte (Safford et al., (1998) Carbohydrate Polymers 35: 155-168). Native Stärken mit hohem Phosphorylierungsgrad verlieren ihre Kristallinität normalerweise bei deutlich niedrigeren Temperaturen als vergleichbare Stärken mit geringem Phosphatgehalt.

Bei vielen thermischen Prozessierungen und Applikationen ist der Einsatz von granulären Reisstärken jedoch wünschenswert. Besonders vorteilhaft ist daher die überraschend hohe DSC T-onset bzw. T-peak Temperatur der erfindungsgemäßen Reisstärken bzw. die überraschend hohe Verkleisterungstemperatur bei der RVA-Analyse der erfindungsgemäßen Reismehle, da aufgrund dieser Eigenschaft der Strukturerhalt der Stärkegranuli bei erhöhten Prozesstemperaturen möglich wird.

In einer weiteren Ausführungsform ist die erfindungsgemäße Reisstärke dadurch gekennzeichnet, dass sie einen Phosphatgehalt in C6-Position (C-6-P) zwischen 0,70 und 2,5 nmol Phosphat pro Milligramm hydrolysierter Stärke aufweist.

Unter dem Begriff "Phosphatgehalt von Stärke" sollen im Zusammenhang mit der vorliegenden Erfindung kovalent an die Glucosemonomere der Stärke gebundene Phosphatgruppen verstanden werden.

Unter dem Begriff "Phosphatgehalt in C6-Position" ist im Zusammenhang mit der vorliegenden Erfindung der Gehalt an Phosphatgruppen zu verstehen, die an Kohlenstoffatom "6" der Glucosemonomere der Stärke gebunden sind. Grundsätzlich können in der Stärke in vivo die Positionen C3 und C6 der Glucoseeinheiten phosphoryliert sein.

Die Bestimmung des Phosphatgehaltes in C6-Position (=C-6-P-Gehalt) erfolgt im Zusammenhang mit der vorliegenden Erfindung über eine Glucose-6-Phosphat-Bestimmung mittels des weiter unten beschriebenen optisch-enzymatischen Tests (Bestimmung des Phosphatgehaltes in C6-Position (C6-P-Gehalt).

In einer bevorzugten Ausführungsform weist die erfindungsgemäße Reisstärke einen Phosphatgehalt in C6-Position zwischen 0,9 und 2,3 nmol Phosphat pro Milligramm Stärke auf. In einer besonders bevorzugten Ausführungsform beträgt der Phosphatgehalt in C6-Position zwischen 1,5 bis 2,0 nmol Phosphat pro Milligramm Stärke.

Die Erhöhung des Phosphatgehaltes von Stärken erfolgt im Zusammenhang mit der vorliegenden Erfindung *in vivo,* nicht in *vitro,* wie beispielsweise durch chemische Phosphorylierung bereits extrahierter Stärke. Daher hat die vorliegende Erfindung den Vorteil, dass chemische Agenzien zur chemischen Phosphorylierung eingespart werden können.

Die vorliegende Erfindung umfasst des weiteren derivatisierte Reisstärke, enthaltend die erfindungsgemäße Reisstärke.

Der Begriff "derivatisierte Reisstärke" bedeutet im Zusammenhang mit der vorliegenden Erfindung eine erfindungsgemäße Reisstärke, deren Eigenschaften nach der Isolierung aus pflanzlichen Zellen mit Hilfe von z.B. thermischen, chemischen, enzymatischen oder mechanischen Verfahren verändert wurden.

Die erfindungsgemäßen Reisstärken sind als Ausgangssubstanz besser geeignet zur Herstellung von derivatisierten Reisstärken als herkömmliche Stärken, weil sie durch den höheren Gehalt an Stärkephosphat einen höheren Anteil an reaktiven funktionalen Gruppen aufweisen und weil die erfindungsgemäße Reisstärke eine höhere Verkleisterungstemperatur bzw. eine höhere thermische Stabilität aufweist als Stärken vergleichbaren Phosphatgehaltes.

Gegenstand der vorliegenden Erfindung sind daher auch Verfahren zur Herstellung einer erfindungsgemäßen derivatisierten Reisstärke, worin die erfindungsgemäße Reisstärke nachträglich modifiziert wird.

Bei der erfindungsgemäßen derivatisierten Reisstärke handelt es sich insbesondere um mit Hitze und/oder mit Säure behandelte Stärke.

In einer weiteren Ausführungsform handelt es sich bei den derivatisierten Reisstärken um Stärkeether, insbesondere um Stärke-Alkylether, O-Allylether, Hydroxylalkylether, O-Carboxylmethylether, stickstoffhaltige Stärkeether, phosphathaltige Stärkeether oder schwefelhaltige Stärkeether.

In einer weiteren Ausführungsform handelt es sich bei den derivatisierten Reisstärken um vernetzte Stärken.

In einer weiteren Ausführungsform handelt es sich bei den derivatisierten Reisstärken um Stärke-Pfropf-Polymerisate.

In einer weiteren Ausführungsform handelt es sich bei den derivatisierten Reisstärken um oxidierte Stärken.

In einer weiteren Ausführungsform handelt es sich bei den derivatisierten Reisstärken um Stärkeester, insbesondere um Stärkeester, die unter Verwendung von organischen Säuren in die Stärke eingeführt wurden. Besonders bevorzugt handelt es sich um Phosphat-, Nitrat-, Sulfat-, Xanthat-, Acetat- oder Citratstärken.

Die erfindungsgemäßen derivatisierten Reisstärken eignen sich für verschiedene Verwendungen in der Pharmaindustrie, im Nahrungsmittel- und/oder Nicht-Nahrungsmittelbereich. Verfahren zur Herstellung von erfindungsgemäßen derivatisierten Stärken sind dem Fachmann bekannt und in der allgemeinen Literatur ausreichend beschrieben. Eine Übersicht zur Herstellung von derivatisierten Stärken findet sich z.B. bei Orthoefer (in Corn, Chemistry and Technology, 1987, eds. Watson und Ramstad, Chapter 16, 479-499). Die Derivatisierung von Reisstärken ist beispielsweise auch beschrieben bei Shih und Daigle (2003), Nahrung 47(1): 64-67.

Ferner ist die Verwendung erfindungsgemäßer Reisstärken zur Herstellung von derivatisierter Stärke Gegenstand der vorliegenden Erfindung.

Die erfindungsgemäße Reisstärke eignet sich in nativer oder derivatisierter Form für verschiedene Verwendungen im Nahrungsmittel- oder Nicht-Nahrungsmittelbereich. Eine weitere Ausführungsform umfasst die Verwendung der erfindungsgemäßen derivatisierten Reisstärke im industriellen Bereich.
Aufgrund der geringen Korngröße der erfindungsgemäßen Reisstärke eignet sich diese besonders auch für die Herstellung von Fotopapieren.

Die vorliegende Erfindung umfasst weiterhin eine Zusammensetzung, enthaltend die erfindungsgemäße Reisstärke.

In einer weiteren Ausführungsform umfasst die vorliegende Erfindung Reismehl, welches die erfindungsgemäße Reisstärke enthält.

In einer weiteren Ausführungsform weisen die erfindungsgemäßen Reismehle DSC T-onset Temperaturen zwischen 72°C und 81°C auf, bevorzugt zwischen 74°C und 80°C. Besonders bevorzugt ist ein erfindungsgemäßes Reismehl, dessen DSC T-onset Temperatur zwischen 76°C und 79°C liegt.

In einer weiteren Ausführungsform weisen die erfindungsgemäßen Reismehle DSC T-peak Temperaturen zwischen 81° und 90°C auf, bevorzugt zwischen 82° und 86°C. Besonders bevorzugt ist ein erfindungsgemäßes Reismehl, dessen DSC T-peak Temperatur zwischen 82°C und 85°C liegt.

In einer weiteren Ausführungsform weisen die erfindungsgemäßen Reismehle in der RVA- (RapidViscoAnalyser) Analyse eine Verkleisterungstemperatur RVA PT zwischen 75 und 90 °C, bevorzugt zwischen 78°C und 88°C und besonders bevorzugt zwischen 80°C und 85°C auf.
Der Begriff "Verkleisterungstemperatur" (RVA PT = RVA Pasting Temperatur) bedeutet im Zusammenhang mit der vorliegenden Erfindung, dass der Messwert für den Beginn der Viskositätsentwicklung gemäß RVA-Analysen diejenige Temperatur ist, bei der die Viskositätskurve während des Aufheizungsprozesses die Basislinie verlässt und bei der sich in einem Zeitintervall von 0,1 Minuten die Viskosität um mehr als 36cP ändert.
Die Bestimmung der "Verkleisterungstemperatur RVA PT" erfolgt im Zusammenhang mit der vorliegenden Erfindung mit Hilfe der unten beschriebenen Methode "Analyse von Reismehl mittels Rapid Visco Analyser (RVA)".
Das erfindungsgemäße Reismehl weist eine erhöhte Verkleisterungstemperatur (RVA PT = RVA Pasting Temperatur) im Vergleich zu Reismehlen aus entsprechenden Wildtyp-Reispflanzen auf.

Der Begriff "erhöhte Verkleisterungstemperatur (RVA PT)" bedeutet im Zusammenhang mit der vorliegenden Erfindung, dass die Verkleisterungstemperatur (RVA PT) um 5°C bis 15°C, insbesondere um 6°C bis 14°C, bevorzugt um 8°C bis 12°C erhöht ist im Vergleich zur Verkleisterungstemperatur RVA PT von Reismehlen aus entsprechenden Wildtyp-Reispflanzen.

In einer weiteren Ausführungsform weist das erfindungsgemäße Reismehl einen verringerten Zeitraum zwischen Erreichen der Pasting Temperatur und Erreichen der Peak Viskosität auf.

Der Begriff "verringerter Zeitraum zwischen Erreichen der Pasting Temperatur und Erreichen der Peak Viskosität" (Peak Time - Pasting Time) bedeutet im Zusammenhang mit der vorliegenden Erfindung, dass die zeitliche Differenz gemäß RVA-Analysen nach der unten beschriebenen Methode 40 sec bis 130 sec, besonders bevorzugt 50 - 100 sec und besonders bevorzugt 60 - 75 sec beträgt.

In einer weiteren Ausführungsform betrifft die vorliegende Erfindung ein Verfahren zur Herstellung eines erfindungsgemäßen Reismehls, worin erfindungsgemäße Reiskörner, vorzugsweise polierte erfindungsgemäße Reiskörner, gemahlen werden. Verfahren zum Polieren und Mahlen von Reiskörnern sind dem Fachmann bekannt und beispielsweise beschrieben in Fitzgerald et al. (2003, J. of Agrocult. And Food Chemistry 51: 2295-2299) oder in Ramesh et al (1999, Carbohydrate Polymers 38: 337-347).

Unter einem "Reiskorn" versteht der Fachmann die ausgereifte, befruchtete Blüte einer Reispflanze.

Die Verwendung eines erfindungsgemäßen Reismehls zur Herstellung von Lebensmitteln und/oder Tierfutter ist ebenfalls von der vorliegenden Erfindung umfasst.

Ein weiterer Gegenstand der vorliegenden Erfindung betrifft eine Zusammensetzung, die das erfindungsgemäße Reismehl enthält.

In einer weiteren Ausführungsform umfasst die vorliegende Erfindung ein Reiskorn, welches die erfindungsgemäße Reisstärke enthält.

In einer bevorzugten Ausführungsform handelt es sich um ein prozessiertes Reiskorn. Unter Prozessierung versteht der Fachmann die Überführung von rohem (Paddy) Reis in braunen oder weißen Reis; Verfahren zur Prozessierung sind dem Fachmann bekannt und u.a. beschrieben in Fitzgerald et al. (2003, J. of Agrocult. and Food Chemistry 51: 2295-2299) oder in Ramesh et al (1999, Carbohydrate Polymers 38: 337-347).

In einer weiteren Ausführungsform der vorliegenden Erfindung lässt sich das erfindungsgemäße Reiskorn zum Kochen verwenden.

In einer weiteren Ausführungsform ist das Reiskorn dadurch gekennzeichnet, dass es veränderte Eigenschaften nach dem Kochen aufweist.

Unter "veränderten Eigenschaften nach dem Kochen" soll im Zusammenhang mit der vorliegenden Erfindung verstanden werden, dass diejenigen Eigenschaften des Reiskornes verändert sind, die die Qualität betreffen, wie z.B. die Textur oder Kornhärte sowie die Klebrigkeit der Körner nach dem Kochen, insbesondere nach Abkühlen bzw. nach Wieder-Erwärmung des gekochten Reises.

In einer bevorzugten Ausführungsform sind die erfindungsgemäßen Reiskörner dadurch gekennzeichnet, dass sie eine Klebrigkeit von -10 bis -200g aufweisen, bevorzugt von -12g bis -150g, besonders bevorzugt von -15g bis -130g (gemessen in g Zugkraft).

Unter dem Begriff "Klebrigkeit" versteht der Fachmann die Auswirkung der Wasseraufnahme und des Erhitzens während des Kochens auf die Konsistenz der Reiskörner und die damit verbundenen Haftung der Reiskörner an anderen Reiskörnern oder anderen Oberflächen (z.B. Gabel, Essstäbchen etc.) nach dem Kochvorgang. Im Zusammenhang mit der vorliegenden Erfindung soll unter Klebrigkeit die maximale negative Kraft (Zugkraft) verstanden werden, welche mittels Texture Analyser nach vorheriger Kompression der optimal gekochten Reiskörner gemessen wird, wie unten in der Methode "Bestimmung der Kocheigenschaften sowie der Textur von gekochten Reiskörnern" beschrieben. Ein hoher negativer Wert bedeutet hierbei eine höhere Klebrigkeit als ein niedrigerer negativer Wert. Unter "optimal gekochten Reiskörnern" sollen im Zusammenhang mit der vorliegenden Erfindung Reiskörner verstanden werden, die nach Erreichen der Minimal-Kochzeit (wenn 90% der Körner kein weißes Zentrum mehr zeigen, wie anhand des Glasplattentestes von Juliano 1984; J. of Tex. Studies 15: 357-376 ermittelt) weitere zwei Minuten gekocht werden.

Bei der vorliegenden Erfindung erfolgt die Messung der Klebrigkeit an optimal gekochten Reiskörnern, an Reiskörnern, die für 22 Stunden bei 4°C gelagert und wieder auf Raumtemperatur gebracht wurden oder an Reiskörnern, die nach Lagerung bei 4°C (für 22 Stunden) mittels eines Ofens bei 80°C für 5 Minuten oder die nach Lagerung bei 4°C (für 22 Stunden) mit Hilfe einer Mikrowelle (600W/ 3 min) wiedererwärmt wurden.

Unter Wiedererwärmen versteht der Fachmann das 1- bis 4-malige Wiedererwärmen des gekochten und zwischenzeitlich abgekühlten Reises, z.B. durch Stehenlassen bei Raumtemperatur, mit Hilfe einer Mikrowelle, eines Ofens, eines Wasserbades oder eines Wärmeschrankes; bevorzugt ist darunter das einmalige Wiedererwärmen zu verstehen.

Da "Klebrigkeit" ein wichtiger Qualitätsparameter für Reis ist, bieten die erfindungsgemäßen Reiskörner, vorteilhafte Einsatzmöglichkeiten. Dazu gehört vor allem der Bereich der Anwendung in Halb-Fertigprodukten, die bei Herstellung lediglich angekocht, anschließend wieder getrocknet und erst kurz vor dem endgültigen Verzehr (z.B. im Privathaushalt, Kantinen) nochmals erhitzt oder aufgekocht werden, ohne dass es zu einer Beeinträchtigung von Geschmack und Konsistenz kommen darf.

Ein weiterer Qualitätsparameter in diesem Zusammenhang ist die Verlängerung der Korndimension beim Kochen in Wasser in Richtung der Längsachse. Eine solche Formveränderung ist ein wichtiger optischer Qualitätsparameter, insbesondere bei Langkornreis.

In einer weiteren Ausführungsform der vorliegenden Erfindung zeichnen sich die erfindungsgemäßen Reiskörner dadurch aus, dass sie eine Elongationsrate (ER) von 1,50 bis 1,90, besonders bevorzugt von 1,55 bis 1,80 und insbesondere bevorzugt von 1,60 bis 1,70 aufweisen.

In einer weiteren Ausführungsform der vorliegenden Erfindung weisen die erfindungsgemäßen Reiskörner eine erhöhte Elongationsrate im Vergleich zu Reiskörnern von entsprechenden Wildtyp- Reispflanzen auf.

Unter einer erhöhten Elongationsrate soll im Zusammenhang mit der vorliegenden Erfindung eine Erhöhung der Elongationsrate um 5% bis 25%, vorzugsweise um 10% bis 20%, besonders bevorzugt um 14% bis 18% verstanden werden.

Unter "Elongationsrate" ist in diesem Zusammenhang das Verhältnis von Kornlänge des gekochten Reiskornes zur Kornlänge des ungekochten Reiskornes zu verstehen. Die Kornlänge wird in mm gemessen, wie beispielsweise in der Methode "Messung der Veränderung der Korndimensionen durch Kochen" beschrieben, vorzugsweise erfolgt die Messung mit einer Schieblehre.

In einer weiteren Ausführungsform weisen die erfindungsgemäßen Reiskörner einen CDC-Wert von 2,8 bis 6,2 auf, bevorzugt von 3,5 bis 5,5 und besonders bevorzugt von 4,0 bis 5,0.

Unter dem "CDC-Wert" (CDC = Coefficient of dimensional changes = Koeffizient der Größenverhältnisse) ist das Verhältnis der kochbedingten Längen- zur Breitenausdehnung zu verstehen, d.h., das Verhältnis der Kornlänge im gekochten (Lc) zum ungekochten (Lu) Zustand in Relation gesetzt zum Verhältnis der Kornbreite in gekochtem (Bc) zum ungekochten (Bu) Zustand = (Lc/Lu)/(Bc/Bu).

Ein weiterer Gegenstand der vorliegenden Erfindung betrifft eine Zusammensetzung, enthaltend mindestens ein erfindungsgemäßes Reiskorn.

Die vorliegende Erfindung umfasst weiterhin eine Reispflanze, tragend mindestens ein erfindungsgemäßes Reiskorn und/oder enthaltend die erfindungsgemäße Reisstärke.

In einer weiteren Ausführungsform umfasst die vorliegende Erfindung genetisch modifizierte Reispflanzenzellen und -pflanzen, die dadurch gekennzeichnet sind, dass die genetische Modifikation zur Erhöhung der Aktivität der löslichen Stärkesynthase II (SSII) führt im Vergleich zu entsprechenden genetisch nicht modifizierten Wildtyp-Reispflanzen bzw. Wildtyp-Reispflanzenzellen.

Unter dem Begriff "lösliche Stärkesynthase II" ist im Zusammenhang mit der vorliegenden Erfindung die Klasse II von löslichen Stärkesynthase-Proteinen (ADP-Glucose 1,4-α-D-glukan 4-α-D-glucosyltransferase; EC 2.4.1.21) zu verstehen. Lösliche Stärkesynthasen katalysieren eine Glycosylierungsreaktion, bei der Glucosereste des Substrates ADP-Glucose unter Bildung einer α-1,4-Verknüpfung auf α-1,4-verknüpfte Glukanketten übertragen werden (ADP-Glucose + {(1,4)- α-D-glucosyl}(N) <=> ADP + {(1,4)- α-D-glucosyl}(N+1)).

SSII-Proteine weisen in ihrem Aufbau eine Abfolge bestimmter Domänen auf. SSII-Proteine haben am "N"-Terminus ein Signalpeptid für den Transport in Plastiden. In Richtung C-Terminus folgen eine N-terminale Region und eine katalytische Domäne (Li et al., 2000, Plant Physiology 123, 613-624). Weitere Analysen, basierend auf Primärsequenzvergleichen (http://hits.isb-sib.ch/cgi-bin/PFSCAN) verschiedener SSII-Proteine ergaben, dass SSII-Proteine drei spezifische Domänen aufweisen. Diese Domänen umfassen die Aminosäuren, die von den Nukleotiden codiert werden: den bp 1190 bis 1279 (= Region 1), den bp 1493 bis 1612 (Region 2) und den bp 2147 bis 2350 (Region 3) der in Seq ID Nr. 1 dargestellten Sequenz des SSII-Gens aus Weizen.

Unter einem SSII-Protein soll daher im Zusammenhang mit der vorliegenden Erfindung eine lösliche Stärkesynthase verstanden werden, deren Aminosäuresequenz mit der unter Seq ID No. 3 angegebenen Region 1 eine Identität von mindestens 86%, bevorzugt mindestens 93% und besonders bevorzugt 100% und mit der unter Seq. ID No. 4 angegebenen Region 2 eine Identität von mindestens 83%, bevorzugt mindestens 86% und besonders bevorzugt 100% und mit der unter Seq. ID No. 5 angegebenen Region 3 eine Identität von mindestens 70%, vorzugsweise mindestens 82%, bevorzugt 86%, besonders bevorzugt 98% und insbesondere bevorzugt von 100% aufweist.

Unter dem Begriff "Identität" soll im Zusammenhang mit der vorliegenden Erfindung der Anteil der übereinstimmenden Aminosäuren (Identität) mit Aminosäuren anderer Proteine verstanden werden, ausgedrückt in Prozent. Bevorzugt wird die Identität mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., (1994) Nucleic Acids Research 22: 4673-4680) ermittelt.

ClustalW wird öffentlich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D - 69117 Heidelberg. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/), sowie bei allen gespiegelten Internetseiten des EBI (European Bioinformatics Institute, Wellcome Trust Genome Campus, Hinxton, Cambridge CB10 1SD, UK), heruntergeladen werden.

Vorzugsweise wird das ClustalW Computerprogramm der Version 1.8 benutzt, um die Identität zwischen hierin beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen den Nukleotidsequenzen der hierin beschriebenen Nukleinsäuremoleküle und der Nukleotidsequenz von anderen Nukleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

Der Begriff "Erhöhung der SSII-Aktivität" bedeutet im Zusammenhang mit der vorliegenden Erfindung eine Erhöhung der SSII-Aktivität im Vergleich zu entsprechenden nicht genetisch modifizierten Wildtyp-Reispflanzen bzw. Wildtyp-Reispflanzenzellen um mindestens 100%, insbesondere um 200% - 2000%, bevorzugt um 400% -1400% und besonders bevorzugt um 500%-900%. Im Zusammenhang mit der vorliegenden Erfindung wird die Aktivität der SSII mit der unten beschriebenen Methode ("Bestimmung der SSII-Aktivität mittels Aktivitätsgel") nachgewiesen. Eine "Erhöhung der SSII-Aktivität" bedeutet im Zusammenhang mit der vorliegenden Erfindung auch, dass Pflanzen oder Pflanzenzellen, die keine nachweisbare SSII-Aktivität aufweisen, nach erfindungsgemäßer genetischer Modifikation, bei der ein fremdes Nukleinsäuremolekül codierend eine lösliche Stärkesynthase in das Genom einer Pflanzenzelle eingeführt wird, eine nachweisbare SSII-Aktivität aufweisen.

Weiterhin überraschend war, dass die erfindungsgemäße Reisstärke eine veränderte Verteilung bestimmter Seitenketten zeigt im Vergleich zu Reisstärke aus entsprechenden Wildtyp-Pflanzenzellen bzw. -Pflanzen.

In einer weiteren Ausführungsform der vorliegenden Erfindung zeigte sich, dass der Gehalt der Seitenketten des Amylopektins mit einem dp (= Degree of Polymerisation) von 20 bis 25 um 5-35% erhöht ist im Vergleich zu Reisstärke aus entsprechenden Wildtyp-Reispflanzen.

Der Begriff "Erhöhung des Gehalts der Seitenketten des Amylopektins mit einem dp (= Degree of Polymerisation) von 20 bis 25" bedeutet im Zusammenhang mit der vorliegenden Erfindung eine Erhöhung des Gehalts an Seitenketten des Amylopektins mit einem DP von 20-25 um 5%-35%, bevorzugt um 6%-30%, besonders bevorzugt um 8%-24% im Vergleich zum Gehalt an Seitenketten mit einem DP von 20-25 von Amylopektin aus entsprechenden Wildtyp- Reispflanzenzellen bzw. -Pflanzen.

In einer weiteren Ausführungsform der Erfindung ist der Gehalt der Seitenketten des Amylopektins mit einem dp von 6 bis 10 verringert.

Der Begriff "Verringerung des Gehalts der Seitenketten des Amylopektins mit einem dp von 6 bis 10" bedeutet im Zusammenhang mit der vorliegenden Erfindung eine Verringerung des Gehalts an Seitenketten des Amylopektins mit einem DP von 6-10 um 20% bis 60%, bevorzugt um 25% bis 55% und besonders bevorzugt um 30% bis 55% im Vergleich zum Gehalt an Seitenketten mit einem DP von 6-10 von Amylopektin aus entsprechenden Wildtyp-Reispflanzenzellen bzw. -Pflanzen.

Die Bestimmung der Seitenkettenverteilung erfolgt im Zusammenhang mit der vorliegenden Erfindung nach der weiter unten beschriebenen Methode ("Aufbereitung von Reismehl/-stärke zur Untersuchung der Amylopektin-Seitenkettenverteilung mittels Hochdruck-Anionenaustausch-Chromatographie"). Die Bestimmung des Anteils an kurzen Seitenketten erfolgt über die Bestimmung des prozentualen Anteils einer bestimmten Seitenkette am Gesamtanteil aller Seitenketten. Der Gesamtanteil aller Seitenketten wird ermittelt über die Bestimmung der Gesamtfläche unter den Peaks, die im HPLC-Chromatogramm die Polymerisationsgrade von DP 6 bis 48 repräsentieren. Der prozentuale Anteil einer bestimmten Seitenkette am Gesamtanteil aller Seitenketten wird ermittelt über die Bestimmung des Verhältnisses der Fläche unter dem Peak, der diese Seitenkette im HPLC-Chromatogramm repräsentiert, zur Gesamtfläche. Zur Bestimmung der Peakflächen kann beispielsweise das Programm Chromelion 6.60 der Firma Dionex, USA, verwendet werden.

Der Begriff "Wildtyp-Reispflanze" bedeutet im Zusammenhang mit der vorliegenden Erfindung, dass es sich um Reispflanzen handelt, die als Ausgangsmaterial für die erfindungsgemäßen Reispflanzen dienen, d.h. deren genetische Information, abgesehen von der eingeführten genetischen Modifikation, die zu einer Erhöhung der Aktivität einer löslichen Stärkesynthase II (SSII) führt, derjenigen einer genetisch modifizierten Reispflanze entspricht.

Vorzugsweise bezieht sich der Begriff "Wildtyp-Reispflanze" auf die Varietät M202, von welcher Körner bei der Hinterlegungsstelle NCIMB Ltd., Ferguson Building, Craibstone Estate, Bucksburn, Aberdeen, Scotland, AB21 9YA, United Kingdom am 17. November 2005 unter der Nummer NCIMB 41352 hinterlegt wurden.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend", dass die Pflanzen, die miteinander verglichen werden, unter gleichen Kulturbedingungen aufgezogen wurden und dass sie vorzugsweise das gleiche (Kultur-) Alter aufweisen.

Unter dem Begriff "Kulturalter" ist die Zeitdauer zu verstehen, die ein (pflanzlicher) Organismus in einem Nährmedium verbringt/wächst. Dies kann z.B. der Zeitraum von Aussaat bis Ernte als auch der Zeitraum der Kultivierung pflanzlicher Zellen in einem Gewebekulturmedium bis zu einem bestimmten Entwicklungsstadium sein. Im Zusammenhang mit der vorliegenden Erfindung ist damit gemeint, dass die Pflanzen und Pflanzenzellen, die miteinander verglichen werden, den gleichen Entwicklungszeitraum unter denselben Kulturbedingungen verbringen.

In einer weiteren Ausführungsform sind die erfindungsgemäßen Reispflanzen und die erfindungsgemäßen Reispflanzenzellen dadurch gekennzeichnet, dass die genetische Modifikation in der Einführung und Expression eines fremden Nukleinsäuremoleküls in den Reispflanzenzellen bzw. den Reispflanzen besteht.

Unter dem Begriff "fremdes Nukleinsäuremolekül" ist im Zusammenhang mit der vorliegenden Erfindung ein solches Molekül zu verstehen, das entweder natürlicherweise in entsprechenden Wildtyp-Reispflanzen bzw. -zellen nicht vorkommt oder das in der konkreten räumlichen Anordnung nicht natürlicherweise in entsprechenden Wildtyp-Reispflanzen bzw. -zellen vorkommt oder das an einem Ort im Genom der Pflanzenzelle lokalisiert ist, an dem es natürlicherweise nicht vorkommt. Bevorzugt ist das fremde Nukleinsäuremolekül/Polynukleotid ein rekombinantes Molekül, das aus verschiedenen Elementen besteht, deren Kombination oder spezifische räumliche Anordnung natürlicherweise in pflanzlichen Zellen nicht auftritt. Dies lässt sich beispielsweise mit Hilfe einer Southern Blot-Analyse nachprüfen.

Im Zusammenhang mit der vorliegenden Erfindung umfasst die genetische Modifikation vorzugsweise das Einführen mindestens eines fremden Nukleinsäuremoleküls, kodierend eine lösliche Stärkesynthase II (SSII), in das Genom einer Pflanzenzelle, wobei besagtes Einführen mindestens eines fremden Nukleinsäuremoleküls dazu führt, dass eine erfindungsgemäße Reisstärke synthetisiert wird.

Für die Einführung von DNA in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten.

Die Transformation monokotyledoner Pflanzen mittels auf Agrobakterium-Transformation basierender Vektoren wurde beispielsweise beschrieben in Chan et al., 1993, Plant Mol. Biol. 22: 491-506; Hiei et al., 1994, Plant J. 6: 271-282; Deng et al., 1990, Science in China 33: 28-34; Wilmink et al., 1992, Plant Cell Reports 11: 76-80; May et al., 1995, Bio/Technology 13: 486-492; Conner und Domisse, 1992, Int. J. Plant Sci. 153: 550-555 und in Ritchie et al, 1993, Transgenic Res. 2: 252-265. Alternative Systeme zur Transformation monokotyledoner Pflanzen sind: die Transformation mittels des biolistischen Ansatzes (Wan und Lemaux, 1994, Plant Physiol. 104: 37-48; Vasil et al., 1993, Bio/Technology 11: 1553-1558; Ritala et al., 1994, Plant Mol. Biol. 24: 317-325; Spencer et al., 1990, Theor. Appl. Genet. 79: 625-631), die Protoplasten-Transformation, die Elektroporation von partiell permeabilisierten Zellen sowie die Einbringung von DNA mittels Glasfasern. Die erfolgreiche Transformation verschiedener Getreidearten wurde beschrieben z.B. für Gerste (Wan und Lemaux, supra; Ritala et al., supra; Krens et al., 1982, Nature 296: 72-74), für Weizen (Nehra et al., 1994, Plant J. 5: 285-297), Reis (Ishida et al., 1996, Nature Biotechnology 14 (6): 745-750) sowie Mais (Koziel et al. (1993), Biotechnology 11: 194-200).

In einer bevorzugten Ausführungsform sind die erfindungsgemäßen Reispflanzen und Reispflanzenzellen dadurch gekennzeichnet, dass es sich bei dem fremden Nukleinsäuremolekül um die kodierende Region einer heterologen löslichen Stärkesynthase II handelt.

Unter einer "heterologen löslichen Stärkesynthase II" ist im Zusammenhang mit der vorliegenden Erfindung eine lösliche Stärkesynthase II zu verstehen, die natürlicherweise nicht in der Pflanzenzelle vorkommt, sondern deren codierende DNA-Sequenz mittels gentechnischer Methoden, wie z.B. Transformation der Zelle, in die Zelle eingeführt wird. Dabei stammt die codierende DNA-Sequenz aus einer anderen Pflanzenart als die transformierte Pflanzenzelle bzw. Pflanze und steht nicht unter der Kontrolle ihres eigenen Promotors. Vorzugsweise stammt die codierende DNA-Sequenz aus einer anderen Pflanzengattung als die transformierte Pflanzenzelle bzw. Pflanze.

Unter dem Begriff "Pflanzengattung" ist im Zusammenhang mit der vorliegenden Erfindung eine hierarchische Stufe der biologischen Systematik zu verstehen. Eine Gattung enthält eine oder mehrere Arten. Ein Beispiel einer Gattung ist *Triticum* L. (Weizen). Alle Arten innerhalb einer Gattung haben immer einen zweiteiligen (binominalen) Namen, der neben der Gattungsbezeichnung noch ein Art-Epipheton enthält. *Triticum aestivum* L. (der Weichweizen) ist danach eine Art der Gattung *Triticum.*

In einer bevorzugten Ausführungsform betrifft die vorliegenden Erfindung Reispflanzen der Gruppe japonica (*Oryza sativa* var. *japonica*), welche Rundkorn- und Mittelkornreissorten umfasst, wie z.B. Klebereis, Milchreis, Mochireis, Nishiki Reis, Ribe Reis, Roter Reis, Schwarzer Reis, Sushi Reis. In einer besonders bevorzugten Ausführungsform der vorliegenden Erfindung werden für die Erzeugung der erfindungsgemäßen Reispflanzen Reis-Wildtyppflanzen der Gruppe japonica als Ausgangsmaterial verwendet.

In einer ganz besonders bevorzugten Ausführungsform umfasst die vorliegende Erfindung genetisch modifizierte Reispflanzen (*Oryza sativa* var. *japonica*) der Transformanten GAOS0353-02301, GAOS0353-01301 und GAOS0353-01502. Saatgut der Transformante GAOS0353-01502 wurde am 17.11.2005 bei der NCIMB Ltd., Ferguson Building, Craibstone Estate, Bucksburn, Aberdeen, Scotland, AB21 9YA, United Kingdom am 17. November 2005 unter der Nummer NCIMB 41353 hinterlegt.

In einer weiteren Ausführungsform weist das erfindungsgemäße Reismehl einen geringeren Gehalt an apparenter Amylose auf als Reismehl aus entsprechenden Wildtyp-Reispflanzen. Die Bestimmung des Gehaltes an apparenter Amylose erfolgt im Zusammenhang mit der vorliegenden Erfindung vorzugsweise mit Hilfe der unten beschriebenen Methode "Bestimmung des Gehaltes an apparenter Amylose". In einer bevorzugten Ausführungsform ist der Gehalt an apparenter Amylose des erfindungsgemäßen Reismehls um 10% bis 20%, besonders bevorzugt um 12% bis 18% und ganz besonders bevorzugt um 13% bis 15% vermindert im Vergleich zum Gehalt an apparenter Amylose in Mehl von entsprechenden Wildtyp-Reispflanzen.

In einer weiteren Ausführungsform der Erfindung steht das eingeführte fremde Nukleinsäuremolekül kodierend eine lösliche Stärkesynthase II unter der Kontrolle eines endospermspezifischen Promotors. Mit Hilfe endospermspezifischer Promotoren ist es möglich, die Transkriptmenge der fremden Nukleinsäuremoleküle kodierend eine lösliche Stärkesynthase II spezifisch im Endosperm der erfindungsgemäßen Pflanzen im Vergleich zum Endosperm entsprechender Wildtyp-Pflanzen zu erhöhen.

Bevorzugt werden Promotoren für eine endospermspezifische Expression verwendet, wie z. B. der Glutelinpromotor aus Reis (Leisy et al. (1990) Plant Mol. Biol. 14: 41-50; Zheng et al. (1993) Plant J. 4:357-366), der HMWG-Promotor aus Weizen (Anderson et al. (1989) Nucleic Acid Res 17:461-462), GBSSII-Promotoren aus Weizen (WO 02/02785), der USP-Promotor (Bäumlein et al. (1991) Mol. Gen Genetics 225: 121-128), der Phaseolinpromotor aus Bohne (Kawagoe und Murai (1992) Plant J 2 (6):927-36), Promotoren von Zein-Genen aus Mais (Pedersen et al., (1982) Cell 29: 1015-1026; Quatroccio et al., (1990) Plant Mol. Biol. 15: 81-93), der shrunken-1-Promotor (sh-1) aus Mais (Werr et al. (1985) EMBO J. 4:1373-1380) oder die Prolamin-Promotoren aus Reis (Qu & Takaiwa (2004) Plant Biotechnology Journal, 2:113-125) verwendet.

Besonders bevorzugt wird der Globulin-Promotor aus Reis (Nakase et al. (1996) Gene 170(2): 223-226) verwendet.

Unter dem Begriff "SSII-Gen" ist im Zusammenhang mit der vorliegenden Erfindung ein Nukleinsäuremolekül oder Polynukleotid (DNA, cDNA) zu verstehen, welches für eine "lösliche Stärkesynthase II" codiert.

In einer weiteren Ausführungsform stammt das SSII-Gen aus einer monokotyledonen Pflanze. In einer bevorzugten Ausführungsform stammt das SSII-Gen aus Weizen.

In einer besonders bevorzugten Ausführungsform weist die lösliche Stärkesynthase II die unter Seq ID No. 2 angegebene Aminosäuresequenz auf.

In einer weiteren Ausführungsform betrifft die vorliegende Erfindung ein Verfahren zur Herstellung einer erfindungsgemäßen Reispflanze, worin aus einer erfindungsgemäßen Reispflanzenzelle eine erfindungsgemäße Reispflanze regeneriert wird und worin nach der Regenration diejenigen Reispflanzen selektiert werden, bei denen die Überexpression der löslichen Stärkesynthase II zu einer Erhöhung der SSII-Aktivität führt.

Der Begriff Selektion bedeutet in Zusammenhang mit der vorliegenden Erfindung, dass ein bewusst ausgewähltes Merkmal in einer Population das Kriterium ist, nachdem Pflanzen, die dieses Merkmal tragen, weiterkultiviert werden, während diejenigen, die das gewünschte Merkmal nicht tragen, verworfen werden.

Im Zusammenhang mit der vorliegenden Erfindung wurden Pflanzen mit dem Merkmal erhöhte SSII-Aktivität selektiert.

Ein weiterer Gegenstand der vorliegenden Erfindung ist das Vermehrungsmaterial der erfindungsgemäßen genetisch modifizierten Reispflanzen, welche die erfindungsgemäßen Reispflanzenzellen enthalten.

Der Begriff "Vermehrungsmaterial" umfasst dabei jene Bestandteile der Pflanze, die geeignet sind zur Erzeugung von Nachkommen auf vegetativem oder sexuellem Weg. Für die vegetative Vermehrung eignen sich beispielsweise Stecklinge oder Kalluskulturen. Vermehrungsmaterial umfasst beispielsweise Früchte, Samen, Sämlinge, Protoplasten, Zellkulturen, etc. Vorzugsweise handelt es sich bei dem Vermehrungsmaterial um endospermhaltige Körner.

Reiskörner von erfindungsgemäßen Reispflanzen, enthaltend genetisch modifizierte Pflanzenzellen, sind ein weiterer Gegenstand der vorliegenden Erfindung.

In einer weiteren Ausführungsform umfasst die vorliegende Erfindung ein Verfahren zur Herstellung einer erfindungsgemäßen modifizierten Reisstärke, umfassend die Extraktion der Stärke aus einer erfindungsgemäßen Reispflanze und/oder aus erfindungsgemäßen Reiskörnern und/oder aus erfindungsgemäßem Reismehl. Methoden zur Extraktion sind dem Fachmann bekannt und z.B. beschrieben bei Wang und Wang (2004, Journal of Cereal. Science 39: 291-296) oder Patindol and Wang (2003, J. Agric Food Chem. 51: 2777-2784).

### Material und Methoden

In den Beispielen wurden die folgenden Methoden verwendet. Diese Methoden können zur Durchführung der erfindungsgemäßen Verfahren verwendet werden, sie stellen konkrete Ausführungsformen der vorliegenden Erfindung dar, beschränken die vorliegende Erfindung jedoch nicht auf diese Methoden. Dem Fachmann ist bekannt, dass er durch Modifikation der beschriebenen Methoden und/oder durch Ersetzen einzelner Methodenteile durch alternative Methodenteile die Erfindung in gleicher Weise ausführen kann.

### 1) Pflanzenmaterial und Kultivierung

Reispflanzen:
*Oryza sativa* Gruppe japonica, Varietät M202
Eine Hinterlegung von Saatgut erfolgte bei der NCIMB Ltd. (National Collection of Industrial Bacteria, Ferguson Building, Craibstone Estate, Bucksburn; Aberdeen, AB21 9YA, UK) am 17.11.2005.
Saatgut der Varietät M202 - Wildtyp erhielt die Hinterlegungsnummer NCIMB 41352; Saatgut der Transformante M202-GAOS0353-01502 die Hinterlegungungsnummer: NCIMB 41353.

Die Kulturführung der Reispflanzen im Gewächshaus erfolgte unter nachfolgenden Bedingungen:
Aussaat: Substrat: Mischung aus 100% Weißtorf mit 100 I Sand/qm sowie Ton:180 kg/qm in Rosentöpfen 1,6 I (Hersteller: H. Meyer, Deutschland). pH: 5,4-6,2;
Gründüngung: Hakaphos (Compo, Deutschland) 14%N -16%P -18%K + 2%Mg; 2kg/qm;
Düngung: 3,5 g/Pflanze bis zur Blüte: NH₄NO₃ (1,75g) und Flory 2 Basis (Hersteller: Euflor, Deutschland): 1,75g; 3%N -16%P - 15%K + 5%Mg.
Temperatur: Tag 28°C / Nacht: 24°C (16h/8h); Rel. Luftfeuchte: 85-95%;
Licht: 16 h, 350µEinstein/s x qm

### 2) Herkunft der zur Transformation verwendeten Sequenzen und Konstrukte

Zur Transformation von Reis wurde die Sequenz Ta_SSIIa aus Weizen verwendet. Isolierung und Klonierung erfolgte wie in WO 97-45545 beschrieben (unter der damaligen Bezeichnung "pTaSS1").
Der verwendete Transformationsvektor AH32-191 ist in Beispiel 1 beschrieben.

### 3) Transformation und Regeneration von Reispflanzen

Reispflanzen wurden nach der von Ishida et al. (1996, Nature BioTechnology, 14 (6): 745-750) beschriebenen Methode transformiert und regeneriert.

### 4) Prozessierung von Reiskörnern

Zur Erzeugung von ausreichenden Mengen an Untersuchungsmaterial wurden Reispflanzen unter Gewächshausbedingungen angezogen und nach Erreichen vollständiger Reife geerntet. Zum weiteren Trocknen werden die reifen (d.h. voll entwickelten) Reiskörner für 3-7 Tage bei 37°C gelagert.
Nachfolgend werden die Körner mittels eines Entspelzers (Laboratory Paddy sheller, Grainman, Miami, Florida, USA) von den Spelzen befreit und der erhaltene braune Reis wird durch 1-minütiges Polieren (Pearlest Rice Polisher, Kett, Villa Park, CA) zu weissem Reis prozessiert. Dieser dient als Ausgangsmaterial für Analysen des ganzen Kornes wie z.B. Alkali spreading value, Korndimension, Korngewicht etc.
Für Untersuchungen der Kornzusammensetzung sowie der Stärke- und Mehleigenschaften werden die weißen Körner mittels einer Labormühle (Cyclotec, Sample mill, Foss, Dänemark) zu Reismehl vermahlen. Das Prinzip der Labormühle beruht darauf, dass das Mahlprodukt die Mahlkammer erst verlässt, wenn eine Partikelgröße von weniger als 0,5 mm erreicht ist. Der Mahlvorgang ist beendet, wenn das gesamte Probenmaterial die Mahlkammer verlassen hat.

### 5) Bestimmung der SSII-Aktivität mittels Aktivitätsgel

Der Nachweis der verschiedenen Stärke-Synthase-Aktivitäten in unreifen Reiskörnern erfolgte mittels Aktivitätsgelen (Zymogrammen), bei denen Proteinextrakte unter nativen Bedingungen in einem Polyacrylamidgel aufgetrennt und nachfolgend mit entsprechenden Substraten inkubiert werden. Das entstandene Reaktionsprodukt (Stärke) wurde mittels Lugol'scher Lösung (2% (w/v) Kl; 0,2% (w/v) I₂) im Gel angefärbt.
Einzelne unreife Reiskörner (ca. 15 Tage nach der Blüte - gemessen ab dem Tag des Blühbeginns) wurden in flüssigem Stickstoff schock gefroren und in 150-200 µl kaltem Extraktionspuffer (50 mM Tris/HCl pH 7,6, 2,5 mM EDTA, 2 mM DTT, 4 mM PMSF, 0,1% (w/v) Glykogen, 10% (v/v) Glyzerin) homogenisiert. Nach Zentrifugation (15 min, 13000 g, 4°C) wurde der klare Überstand in ein frisches Reaktionsgefäss überführt und ein Aliquot des Extraktes zur Bestimmung des Proteingehaltes nach Bradford (1976, Anal Biochem 72: 248-254) verwendet.
Die Auftrennung der Proteinextrakte erfolgte mittels eines kontinuierlichen 7,5% Polyacrylamid-Geles (7,5% AA/BAA 37,5:1; 25 mM Tris/HCl pH 7,6, 192 mM Gylcin, 0,1% (w/v) APS, 0,05% (v/v) TEMED) unter Verwendung von einfach konzentriertem Laufpuffer (25 mM Tris/HCl, 192 mM Glycin). Vor Beladung der Gele erfolgt ein Vorlauf zur Entfernung von Radikalen für 30 Minuten bei 8mA und 4°C. Für jede Probe wurden 15 µg Protein aufgetragen und die Elektrophorese für 2 - 2,5 Stunden bei 4°C durchgeführt.
Nachfolgend wurden die Gele in 15 ml Inkubationspuffer ( 0,5M Natriumcitrat pH 7,0, 25mM Kaliumacetat, 2mM EDTA, 2 mM DTT, 0,1% (w/v) Amylopektin, 50mM Tricine/NaOH pH 8,5, 1 mM ADP-Glucose) über Nacht bei Raumtemperatur unter ständigem Schütteln inkubiert. Die Anfärbung der gebildeten Stärke erfolgte mittels Lugol'scher Lösung.
Zur Ermittlung des Ausmaßes der Erhöhung der SSII-Aktivität mittels Zymmogrammen wurden Proteinextrakte der genetisch modifizierten Linien stufenweise verdünnt und entsprechend der oben beschriebenen Methode verwendet. Nach Anfärbung der Zymogramme mit Lugol'scher Lösung wurde anhand des optischen Vergleiches der Intensität der SSII-Bande für die verschiedenen Verdünnungen mit dem unverdünnten Wildtyp das Ausmaß der Aktivitätserhöhung ermittelt.

### 6) Extraktion von Reisstärke aus Reismehl

Die Extraktion von Reisstärke aus Reismehl erfolgte in Anlehnung an die bei Wang und Wang (2004; Journal of Cereal Science 39: 291-296) beschriebene Methode.
10g Reismehl wurden mit 40ml 0,05% (w/v) NaOH für 16-18 Stunden auf einem Schüttler bei Raumtemperatur inkubiert. Nachfolgend wurde die Suspension zur Vervollständigung des Aufschlusses in einen Warring Blender überführt und für 15 Sekunden bei geringer Geschwindigkeit sowie anschließend 45 Sekunden bei hoher Geschwindigkeit durchmischt. Zur Abtrennung von größeren Bestandteilen (z.B. Zellwand) wurde die Suspension auf einander folgend durch Siebe mit einer Maschengröße von 125µm bzw. 63µm gegeben. Nach Zentrifugation bei 1500 rpm für 15 Minuten (Microfuge 3.OR; Heraeus) wurde der Überstand abgegossen und die an der Oberfläche des Pellets liegende Proteinschicht mit einem Spatel entfernt. Das restliche Pellet wurde nochmals in 0,05 % (w/v) NaOH resuspendiert und der oben beschriebene Vorgang wiederholt. Nachfolgend wurde das Pellet in Wasser resuspendiert und der pH-Wert der Suspension mit HCI auf 6,5-7 eingestellt. Die erhaltene Reisstärke wurde insgesamt dreimal mit Wasser gewaschen, wobei jeder Waschritt Sedimentation (1500 rpm, 15 min, RT), Verwerfen des Überstandes und Resuspension in frischem Wasser umfasste. Vor dem letzten Waschschritt wurde erneut der pH-Wert überprüft und ggf. mit HCl auf pH 7 eingestellt. Das Reisstärke-Pellet des letzten Waschschrittes wurde in Aceton resuspendiert, sedimentiert und der Überstand verworfen. Nach erneuter Resuspendierung des Pellets in Aceton wurde die Suspension in eine Petrischale gegossen und unter dem Abzug für mindestens 18 Stunden bei Raumtemperatur getrocknet.
In einem letzten Schritt wurde die Reisstärke durch Mörsern in ein feines Pulver überführt, welches direkt für alle weiteren Untersuchungen eingesetzt wurde.

### 7) Aufbereitung von Reismehl/-stärke zur Untersuchung der Amylopektin-Seitenkettenverteilung mittels Hochdruck-Anionenaustausch-Chromatographie

Pro Probe wurden 10 mg Reismehl oder-stärke in ein 2 ml Eppendorfcup eingewogen und mit 250µl 90% (v/v) DMSO versetzt. Nach Lösen der Probe unter Schütteln bei 60°C wurden 375µl Wasser zugesetzt und der Ansatz für eine Stunde bei 95°C inkubiert. Zu 200µl des Ansatzes wurden 300µl 16,7 mM Natriumacetat pH 3,5 sowie 0,5 U Isoamylase aus Pseudomonas sp. (Megazyme; Bray, Irland) gegeben. Nach 24 Stunden Inkubation bei 37°C wurden weitere 0,5 U Isoamylase zugesetzt und die Inkubation für weitere 24 Stunden fortgesetzt.

Für die Chromatographie wurden 100 µl des Ansatzes 1:5 mit Wasser verdünnt und nachfolgend durch Ultrafree-MC Filtertubes (Millipore) filtriert. Etwa 90µl des Filtrats wurden injiziert.

### Chromatographie:

### Methode :

- **HPLC- Anlage:**: **GP 50 Dionex Gradient Pump**
ED 50 Dionex Electrochem. Detector/ PAD
AS 50 Autosampler
Säulenofen
- Säule:: Dionex CarboPac PA 100 4 x 250 mm (P/N 046110)
mit Guard Column PA 100 4 x 50 mm (P/N 046115)

### Gerätekon aus Reiskörnern uration:

Eluenten-
reservoir
A / C / D
→
Pumpe
GP50
A / C / D
→
Autosampler
AS50
→
Säulen
Dionex Guard
Column PA 100
4 x 50 mm
↓
Dionex
CarboPac
PA 100
4 x 250 mm
→
PAD
→
Waste

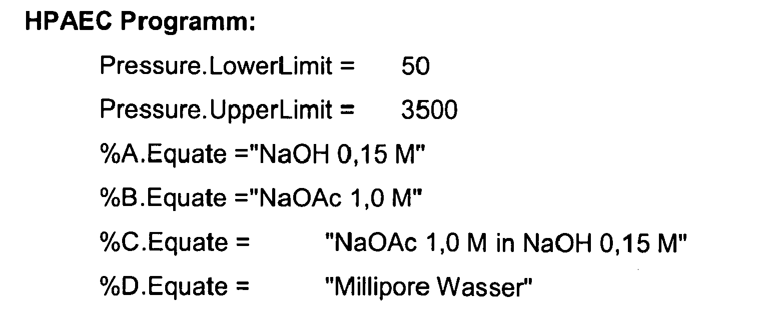

ECD.Data_Collection_Rate = 1.0
Waveform Time = 0.00, Potential = 0.05
Waveform Time = 0.20, Potential = 0.05, Integration = Begin
Waveform Time = 0.40, Potential = 0.05, Integration = End
Waveform Time = 0.41, Potential = 0.75
Waveform Time = 0.60, Potential = 0.75
Waveform Time = 0.61, Potential = -0.15
Waveform Time = 1.00, Potential = -0.15
Cell = On
Flush Volume = 500
Wait FlushState
NeedleHeight = 2
CutSegmentVolume = 10
SyringeSpeed = 4 ;
Cycle = 0
WaitForTemperature = False
Wait SampleReady

0.000 Flow = 1.00
%B = 0.0
%C = 0.0
%D = 0.0
Curve = 5
Load
Inject
Wait
ECD.Autozero
ECD_1.AcqOn
Flow = 1.00
%B = 0.0
%C = 0.0
%D = 0.0
Curve = 5

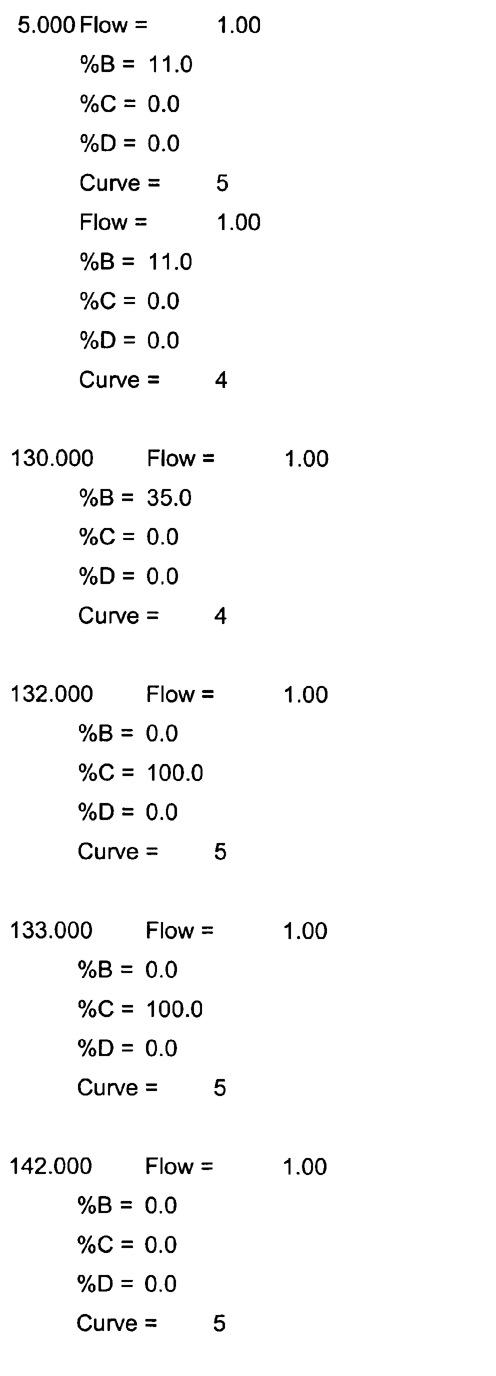

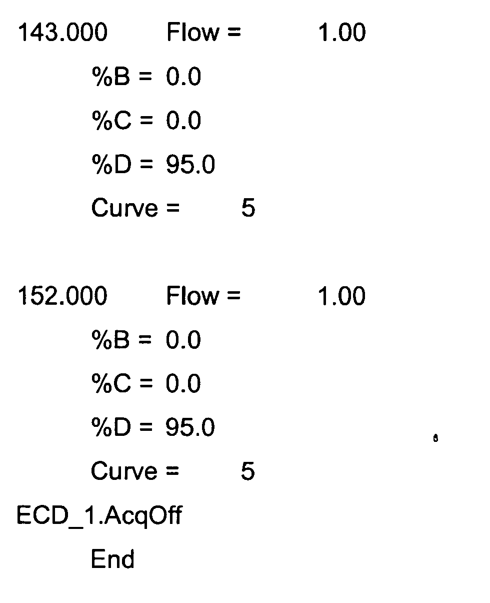

Die Auswertung der Daten erfolgt mit Dionex Chromeleon v6.60.

Zum Vergleich der Chromatogramme gegeneinander wurden für jedes Chromatogramm die identifizierten Peaks der unterschiedlichen Maltooligasaccharide Mittelwert-normalisiert (Summe aller Peakflächen = 1). Die Auswertung erfolgte aufgrund der "force common baseline", wie im Dionex Chromeleon v.6.60 zu "log baseline" beschrieben. Dabei wird die log baseline gesetzt kurz vor dem ersten Seitenkettenpeak und bis zum letzten auswertbare Peak des kürzesten Chromatogramms eines Messdurchgangs, daraus wird der letzte auswertbare Peak für alle Chromatogramme berechnet

### 8) Bestimmung der Kocheigenschaften sowie der Textur von gekochten Reiskörnern

Zur Bestimmung der Kocheigenschaften wurden weiße Reiskörner verwendet, die wie unter 4) "Prozessierung von Reiskörnern" beschrieben, prozessiert wurden. Vor dem Kochen wurden die Korndimensionen sowie das Korngewicht ermittelt. Das Kochen fand in einem Verhältnis von Wasser zu Reis von 20:1 statt.
Das Wasser wurde zum Kochen gebracht, der Reis zugegeben und die Wärmezufuhr soweit reduziert, dass das Wasser leicht köchelte (dabei wurde der Reis alle 3 Minuten umgerührt). Mittels des Glasplatten-Testes von Juliano (1984; J. of Tex. Studies 15: 357-376) wurde die minimale Kochzeit ermittelt. Hierzu wurden im Abstand von einer Minute jeweils 10 Körner zwischen zwei Glasplatten zerdrückt. Zu dem Zeitpunkt, an dem 90% der Körner kein weißes Zentrum mehr zeigten, war die Minimal-Kochzeit erreicht. Durch Verlängerung des Kochprozesses um weitere zwei Minuten wurde die optimale Kochzeit erreicht. Der Reis wurde über ein Sieb abgegossen und bei Raumtemperatur abgekühlt. Nachfolgend wurden erneut die Korndimensionen und das Gewicht der gekochten Körner bestimmt. Die Messung der Textur erfolgt an frisch gekochten Reiskörnern (ca. 1h nach dem Kochen), an Reiskörnern, die für 22 Stunden bei 4°C gelagert und wieder auf Raumtemperatur erwärmt wurden sowie an Reiskörnern, die mittels Ofen bzw. Mikrowelle nach Lagerung bei 4°C (für 22 Stunden) wiedererwärmt wurden. Zum Wiedererwärmen der gekochten Reiskörner im Ofen wurden diese in ein Schälchen aus Alufolie gegeben und dieses zur Vermeidung von Feuchtigkeitsverlusten mit Alufolie verschlossen. Die Inkubation im Ofen erfolgte für 20 Minuten bei 80°C. Das Wiedererwärmen der Körner in der Mikrowelle erfolgte in einem entsprechenden Mikrowellen-Behältnis für drei Minuten bei 360 Watt. Nach beiden Wiedererwärmungsprozessen wurden die Körner für 30 Minuten bei Raumtemperatur gelagert, um eine einheitliche Temperatur der Körner während der Messung zu gewährleisten.
Die Messung der Textur von gekochtem Reis aus den oben beschriebenen Versuchen erfolgte mit einem Texture Analyser TAXT2 (Stable Micro Systems, Godalming, UK) unter Verwendung einer runden Sonde mit einem Durchmesser von 2,5cm und einem doppelten Kompressionstest als Messmethode (in Anlehnung an Champagne et al. (1998) Cereal Chem. 75 (2): 181-186). Hierbei wurden die Reiskörner in einem ersten Zyklus komprimiert, nachfolgend entlastet und erneut komprimiert, wobei die Kraft, die die Körner der Sonde entgegenbringen (Druck oder Zug), kontinuierlich aufgezeichnet wurde. Für jede zu analysierende Probe wurden zehn Messungen von jeweils drei Körnern durchgeführt, wobei die Reiskörner so unter die Messsonde gelegt wurden, dass sie sich weder untereinander berühren noch über den Rand der Messsonde herausstehen. Erfasst wurden die Festigkeit (H) der gekochten Reiskörner (maximale Kraft während der ersten Kompression) sowie die Klebrigkeit (-H) (minimale Kraft nach der ersten Kompression). Alle Messungen einer Probe wurden getrennt ausgewertet und nachfolgend Mittelwerte für die entsprechenden Parameter erstellt.

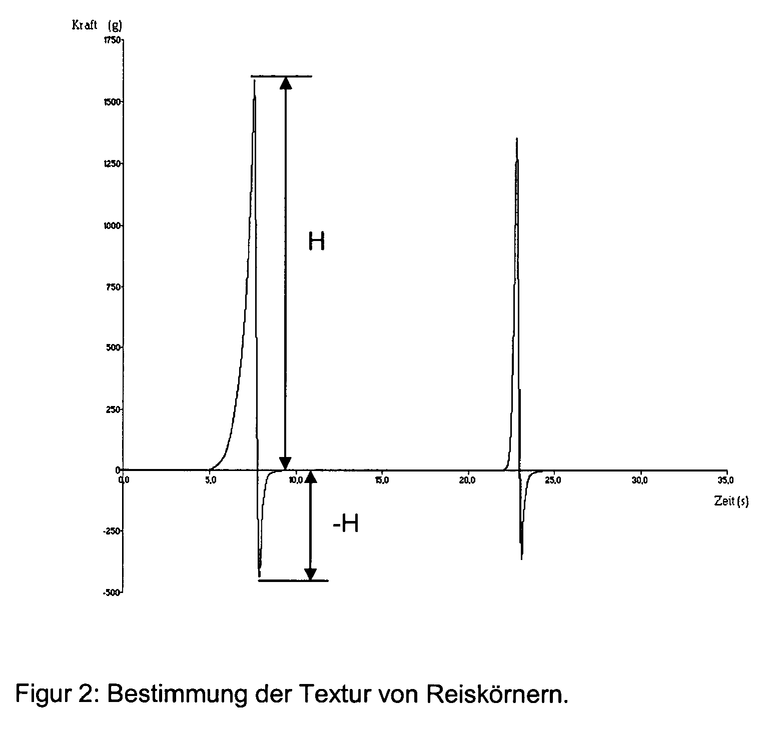

### 9) Messung der Veränderung der Korndimensionen durch Kochen

Die Bestimmung der Korndimensionen (Länge, Breite und Fläche) erfolgte unter Verwendung der Software "SigmaScan Pro" Version 5.0.0 der Firma Systat (Erkrath, Deutschland).
Hierzu wurden jeweils 30 Reiskörner (ungekocht und gekocht) eingescannt und das entstandene Bild mittels der Software ausgewertet. Folgende Parameter wurden erfasst bzw. berechnet:
Lᵤ - Kornlänge ungekochter Reis Bᵤ - Kornbreite ungekochter Reis
   L_{c} - Kornlänge ungekochter Reis B_{c} - Kornbreite ungekochter Reis
L/B-Verhältnis = Lᵤ/Bᵤ bzw. L_{c}/B_{c}
   ER - Elongationsrate = L_{c}/Lᵤ
   CDC - Koeffizient dimensionaler Veränderungen= (L_{c}-Lᵤ)/(B_{c}-Bᵤ)

### 10) Thermische Analyse von Reismehl/-stärke mittels Wärmefluss-Kalorimetrieverfahren (Differential scanning calorimetry (= DSC))

Einwaagen von etwa 10 mg (Trockengewicht) Reismehl oder Reisstärke wurden mit einem Überschuss an bi-destilliertem Wasser (bevorzugt 30 µl) in Edelstahlpfännchen (Perkin Elmer, "Large Volume Stainless Steel Pans" [03190218], Volumen 60 µl) gegeben und mit Hilfe einer Presse hermetisch verschlossen. Mit einer Heizgeschwindigkeit von 10°C/min wurde die Probe von 20°C bis 150°C in einem Diamond DSC-Gerät (Perkin Elmer) erhitzt. Dabei dient ein leeres verschlossenes Edelstahlpfännchen als Referenz. Das System wurde mit definierten Mengen Indium kalibriert.
Die Datenanalyse wurde mittels Softwareprogramm von Pyris (Perkin Elmer, Version 7.0) durchgeführt. Die Weiterverarbeitung auswertbarer Rohdaten erfolgte durch Analyse der einzelnen Peaks der Phasenübergänge 1. Ordnung auf T-onset (°C), T-peak (°C), T-end (°C) und dH (J/g) (Standard ist dabei die gerade Basislinie).
DSC T-onset ist dabei charakterisiert als der Schnittpunkt zwischen der Verlängerung der Basislinie und der an die ansteigende Flanke des Peaks angelegten Tangente durch den Wendepunkt. Sie charakterisiert den Beginn der Phasenumwandlung.
Als Maximaltemperatur DSC T-peak wird die Maximaltemperatur bezeichnet, bei der die DSC-Kurve ein Maximum erreicht hat (d.h., diejenige Temperatur, bei der die erste Ableitung der Kurve Null ist).
Bei der in Pyris verwendeten Funktion (calc-peak Area) wird von Hand eine Start- und eine Endtemperatur für den Baselinefit eingegeben.

### 11) Bestimmung des Gehaltes an apparenter Amlyose

Die Bestimmung des Gehaltes an apparenter Amylose erfolgte in Anlehnung an die Methode von Juliano (1971, Cereal Science Today 16 (10): 334-340).
Für jede Probe wurden zweimal 50 mg Reismehl in 100 ml Erlenmeyerkolben eingewogen und auf einander folgend mit 1 ml 95% Ethanol und 9 ml 1M NaOH befeuchtet.
Parallel werden zur Anfertigung einer Standardkurve Kolben mit definierten Mengen an reiner Amylose in der gleichen Weise behandelt wie die Mehlproben. Die Kolben wurden zur Durchmischung kurz geschwenkt und anschließend für 20 Minuten im kochenden Wasserbad unter leichtem Schütteln inkubiert. Nach 5-10 Minuten Abkühlen bei RT wurde das Volumen mit Wasser auf 100 ml aufgefüllt.

Ein Aliquot von 100µl wurde mit 1 ml Messlösung (10 mM Essigsäure, 0,004% (w/v) I₂; 0,04% (w/v) KI) versetzt, gut durchmischt und die Absorption bei 620nm gegen einen entsprechenden Blindwert bestimmt. Die Berechnung des Amylose-Gehaltes erfolgte mit Hilfe der Amylose-Standards, die zur Erstellung einer Eichkurve verwendet werden.

### 12) Analyse von Reismehl mittels Rapid Visco Analyser (RVA)

Das Prinzip dieser Analyse beruht darauf, dass eine Suspension aus Wasser und Reismehl einem definierten Temperatur- und Scher-Protokol unterzogen wird und dabei kontinuierlich die Viskosität der Suspension aufgezeichnet wird. Als Messgerät dient ein RVA Super3 der Firma Newport Scientific (Macclesfield, UK) mit der entsprechenden Software "Thermocline for Windows", Version 2.3.

Für die Analyse wurden 3g Reismehl (Einwaage als reines Trockengewicht des Probenmaterials, korrigiert auf 0% Feuchte) in ein Messgefäß eingewogen, mit 25 ml Wasser versetzt und das Messgerät nach Einsetzen eines Rührers in das Gerät eingespannt.
Das folgende Temperatur- und Scher-Profil wurde appliziert:

| Zeit | Art | Wert |
|---|---|---|
| 00:00:00 | Temp | 50°C |
| 00:00:00 | Speed | 960 rpm |
| 00:00:10 | Speed | 160 rpm |
| 00:01:00 | Temp | 50°C |
| 00:04:48 | Temp | 95°C |
| 00:07:18 | Temp | 95°C |
| 00:11:06 | Temp | 50°C |
| 00:12:30 | End of test | |

Nach Beendigung der Messung wurden die folgenden Parameter ermittelt:
Peak Viskosität (höchste Viskosität zwischen 2 und 7 Minuten Messzeit)
Trough Viskosität (geringste Viskosität zwischen 7 und 12 Minuten Messzeit)
Final Viskosität (Viskosität am Ende der Messung)
Breakdown = Peak - Trough
Setback = Final - Trough
Pasting Temperatur (Temperatur, bei der sich in einem Zeitintervall von 0,1 Minuten die Viskosität um mehr als 36cP ändert)
Peak time (Zeit, bei der die Peak Viskosität erreicht ist)

### 13) Bestimmung des Phosphatgehaltes in C6-Position (C6-P-Gehalt)

In der Stärke können die Positionen C3 und C6 der Glucoseeinheiten phosphoryliert sein. Zur Bestimmung des C6-P-Gehaltes der Stärke (modifiziert nach Nielsen et al., 1994, Plant Physiol. 105: 111-117) wurden 50 mg Reismehl in 500 µl 0,7 M HCl 4 h bei 95°C unter ständigem Schütteln hydrolysiert. Anschließend wurden die Ansätze für 10 min bei 15.500 g zentrifugiert und die Überstände mittels einer Filtermembran (0,45 µM) von Schwebstoffen und Trübungen gereinigt. Von dem klaren Hydrolysat wurden 20 µl mit 180 µl Imidazol-Puffer (300 mM Imidazol, pH 7,4; 7,5 mM MgCl₂, 1 mM EDTA und 0,4 mM NADP) gemischt. Die Messung wurde im Photometer bei 340 nm durchgeführt. Nach Erfassung der Basisabsorption wurde die Enzymreaktion durch die Zugabe von 2 Einheiten (units) Glucose-6-Phosphat Dehydrogenase (von *Leuconostoc mesenteroides,* Boehringer Mannheim) gestartet. Die Absorptionsänderung beruht auf einer äquimolaren Umsetzung von Glucose-6-Phosphat und NADP zu 6-Phosphorglukonat und NADPH, wobei die Bildung des NADPH bei der o. g. Wellenlänge erfasst wird. Die Reaktion wurde bis zum Erreichen eines Plateaus verfolgt. Das Ergebnis dieser Messung liefert den Gehalt an Glucose-6-Phosphat im Hydrolysat. Aus dem identischen Hydrolysat wurde anhand des Gehaltes an freigesetzter Glucose der Grad der Hydrolyse bestimmt. Dieser wird verwendet, um den Gehalt an Glucose-6-Phosphat auf den Anteil hydrolysierter Stärke aus der Menge Frischgewicht zu beziehen. Hierzu wurden 10µl Hydrolysat mit 10µl 0,7 M NaOH neutralisiert und nachfolgend 1:100 mit Wasser verdünnt. 4 µl dieser Verdünnung wurden mit 196µl Messpuffer (100mM Imidazol pH 6,9; 5 mM MgCl₂, 1 mM ATP, 0,4 mM NADP) versetzt und zur Bestimmung der Basisabsorption verwendet. Die Reaktion wurde durch Zugabe von 2 µl Enzym-Mix (Hexokinase 1:10; Glucose-6-Phosphat Dehydrogenase aus Hefe 1:10 in Messpuffer) und bei 340nm bis zum Plateau verfolgt. Das Messprinzip entspricht dem der ersten Reaktion.

Das Ergebnis dieser Messung liefert die Menge an Glucose (in mg), die im Verlauf der Hydrolyse aus der im Ausgangsmaterial vorhandenen Stärke freigesetzt wurde.
Nachfolgend wird das Ergebnis beider Messung in Relation gesetzt, um so den Gehalt an Glucose-6-Phosphat pro mg hydrolysierter Stärke auszudrücken. Anders als bei einem Bezug der Menge an Glucose-6-Phosphat auf das Frischgewicht der Probe wird durch diese Berechnung die Menge an Glucose-6-Phosphat lediglich auf den Teil der der Stärke bezogen, welcher vollständig zu Glucose hydrolysiert wurde und somit auch als Quelle für das Glucose-6-Phosphat anzusehen ist.

### Beispiele

### Beispiel 1: Transformationsvektor zur Expression einer Stärkesynthase IIa aus Weizen in Reis

Verwendet wurden der Reis-Transformationsvektor IR103-123 (beschrieben in WO 05/030941) und das Plasmid CF31-191 (beschrieben in WO 97/45545 unter der Bezeichnung pTaSS1). Der Reistransformationsvektor IR103-123 dient der endospermspezifischen Expression des Zielgens mittels des Globulin-Promotors aus Reis. In einem ersten Schritt a) erfolgt die Öffnung des Vektors IR103-123 mit den Restriktionsenzymen *EcoRV* und *Xhol.* Das Plasmid CF31-191 enthält die cDNA einer Stärke-Synthase IIa (SSIIa) aus Weizen (*Triticum aestivum*). In einem zweiten Schritt b) wird die cDNA der SSIIa mit den Restriktionsenzymen *Ecl136II* und *Xho*l aus dem Plasmid CF31-191 ausgeschnitten. Die Ligation des in Schritt a) geöffneten Vektors IR103-123 mit dem in Schritt b) erhaltenen Fragment aus dem Plasmid CF 31-191 liefert den Vektor AH32-191.

### Beispiel 2: Herstellung genetisch modifizierter Reispflanzen, die eine erhöhte SSII-Aktivität aufweisen

Zur Erzeugung genetisch modifizierter Pflanzen mit erhöhter Stärkesynthase II (SSII) - Aktivität wurde die T-DNA des Plasmids AH32-191 mit Hilfe von Agrobakterien, wie bei Ishida et al. (1996, Nature Biotechnology 14 (6): 745-750) beschrieben, in Reispflanzen transferiert. Die Erhöhung der SSII-Aktivität wurde mittels Zymogramme ermittelt.

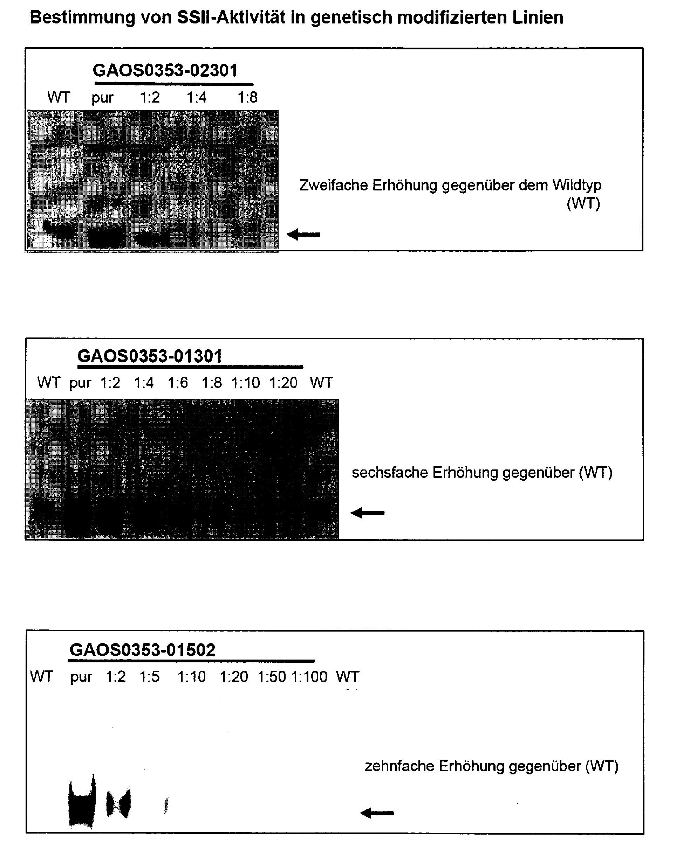

Figur 1 zeigt Zymogramme dreier genetisch modifizierter Reis-Linien zur Bestimmung der SSII-Aktivität im Vergleich zum Wildtyp. Verwendet wurden Gesamtproteinextrakte von unreifen Körnern (15 Tage nach Blühbeginn) vom Wildtyp und den jeweiligen genetisch modifizierten Linien in jeweils gleichen Mengen. Die Proteinextrakte der genetisch modifizierten Linien wurden stufenweise verdünnt und anhand des optischen Vergleiches der Intensität der SSII-Bande in diesen Spuren mit der "Wildtyp-Spur" das Ausmaß der Aktivitätserhöhung ermittelt. Die SSII-Aktivität der Linie GAOS0353-01502 ist zehnmal, der Linie GAOS0353-01301 sechsmal und der Linie GAOS0353-02301 doppelt so hoch wie in den Körnern des Wildtyps.

### Beispiel 3: Zusammenstellung der Eigenschaften von Reiskörnern, Reisstärke und Reismehl verschiedener genetisch modifizierter Linien mit unterschiedlich hoher SSII-Expression

**Tabelle 1: Eigenschaften von Reisstärke aus Reiskörnern mit veränderter SSII-Expression im Vergleich zum Wildtyp.**

| Linie | Expression SSII (xfach WT) | Erhöhter AP-SK Bereich | Reduzierter AP-SK Bereich | C6P (nmol/mg Stärke) | DSC Tₒₙₛₑₜ | DSC T_{Peak} | DSC Tₒₙₛₑₜ (%) | DSC T_{Peak} (%) |
|---|---|---|---|---|---|---|---|---|
| Wildtyp | 1 | x | x | 0,50 | 64,1 | 69,5 | 100 | 100 |
| GAOS0 353-02301 | 2 | 10-26 | 6-9 | 0,74 | 76,2 | 80,8 | 119 | 116 |
| GAOSO 353-01301 | 6 | 11-29 | 6-9 | 0,92 | n.d. | n.d. | n.d. | n.d. |
| GAOS0 353-01502 | 10 | 12-31 | 6-10 | 1,80 | 77,6 | 82,5 | 121 | 119 |

Aufgeführt sind die SSII-Aktivität im unreifen Reiskorn (als Vielfaches des Wildtyps), die Bereiche der Amylopektin-Seitenketten der Stärke (AP-SK), welche gegenüber dem Wildtyp signifikant verändert sind, der Phosphatgehalt (C6P) und die DSC-Werte in °C und in % von Reisstärke (im Vergleich zum Wildtyp), (n.d.= nicht detektiert). Die Phosphatgehalte in C6-Position der Stärken von genetisch modifizierten Linien sind signifikant erhöht im Vergleich zum Wildtyp in Abhängigkeit von der Höhe der SSII-Aktivität.

**Tabelle 2: Eigenschaften von Reismehl aus Reiskörnern mit veränderter SSII-Expression im Vergleich zum Wildtyp.**

| Linie | Expression SSII (xfach WT) | DSC Tₒₙₛₑₜ | DSC T_{Peak} | DSC Tₒₙₛₑₜ (%) | DSC T_{Peak} (%) | Amylose (%) | Amylose (% WT) |
|---|---|---|---|---|---|---|---|
| Wildtyp | 1 | 65,6 | 71,5 | 100 | 100 | 14,0 | 100 |
| GAOS 03 53-02301 | 2 | 77,4 | 82,0 | 118 | 115 | 12,5 | 89 |
| GAOS 03 53-01301 | 6 | 77,6 | 82,5 | 118 | 115 | 12,5 | 89 |
| GAOS 03 53-01502 | 10 | 78,8 | 84,6 | 120 | 118 | 11,7 | 84 |

Aufgeführt sind die SSII-Expression im unreifen Reiskorn (als Vielfaches des Wildtyps), die Änderungen der DSC-Werte von Reismehl, in °C und in Prozent (%) vom Wildtyp.

Die apparenten Amylosegehalte der genetisch modifizierten Linien zeigen nur geringe Veränderungen und liegen sämtlich leicht unter denen des Wildtyps.

Die thermische Stabilität sowohl der Reismehle als auch der daraus isolierten Stärken nehmen graduell mit der Höhe der SSII-Aktivität in den unterschiedlichen genetisch modifizierten Linien zu (Vergleiche Figur 1 und Tabellen 1 und 2). Die stärkste Ausprägung der Erhöhung der DSC T-onset bzw. DSC T-peak zeigt die Linie mit einer 10fach erhöhten SSII-Aktivität. Die Werte liegen jeweils bei ca. 120% bezogen auf den Wildtyp.

### Beispiel 4: Vergleich verschiedener genetisch modifizierter Reis-Linien bezüglich

### Aktivitätshöhe und Amylopektin-Seitenketten im Vergleich zum Wildtyp (WT).

In Tabelle 3 ist die Verteilung der Amylopektin-Seitenketten verschiedener genetisch modifizierter Reis-Linien im Vergleich zum Wildtyp dargestellt. Die dargestellten Kurven ergeben sich aus der Auftragung der Kettenlänge (in DP = Degree of Polymerization) der analysierten Glukane gegen den prozentualen Anteil, den ein bestimmter DP an der Gesamtmenge aller untersuchten DPs aufweist.

**Tab. 3: Verteilung des Seitenkettenprofils von Amylopektin der genetisch modifizierten Linien im Verhältnis zum Wildtyp(WT)-Mehl, eingeteilt in Gruppen mit unterschiedlichem Grad der Polymerisation.**

| Grad der Polymerisation (dp) | % bezogen auf WT-Mehl | | |
|---|---|---|---|
| | GAOS 0353-2301 | GAOS 0253-1301 | GAOS 0253-150225 |
| dp 6-10 | 81,4 | 66,3 | 47,0 |
| dp 20-25 | 108,7 | 115,7 | 123,8 |

Es zeigt sich, dass mit steigender Aktivität der SSII eine abgestufte Veränderung der Amylopektin-Seitenketten Verteilung einhergeht. Mit einer Zunahme der SSII-Aktivität kommt es zu einer graduell unterschiedlich starken Reduktion der Seitenketten mit einem DP zwischen 6-10 sowie einer Zunahme der Seitenketten mit einem DP von 20-25.

### Beispiel 5: Textur von gekochten Reiskörnern

Reiskörner verschiedener genetisch modifizierter Linien sowie des entsprechenden Wildtyps wurden bis zur jeweiligen optimalen Kochzeit (siehe Methode "Bestimmung der Eigenschaften von gekochten Reiskörnern") gekocht. Die Messung der Textur erfolgt an Reiskörnern, die nach dem Kochen für 22h bei 4°C gelagert (Tab. 4a, angegeben sind die Mittelwerte aus 10 Messungen pro Probe (jeweils 3 Körner)). wurden oder an frisch gekochten Reiskörnern (ca. 1h nach dem Kochen) sowie an kalt gelagerten (4°C, 22h) und danach mittels Ofen bzw. Mikrowelle wiedererwärmten Reiskörnern (Tab. 4b).

**Tabelle 4a: Textur von Reiskörnern, die nach dem Kochen für 22h bei 4°C gelagert wurden**

| Probe | Kraft 2 (Klebrigkeit in g) | Kraft1 (Kornhärte in g) |
|---|---|---|
| Mittel_Wildtyp M202 | -239,5 | 1164,6 |
| Mittel_GAOS0353-02301 | -137,0 | 1393,0 |
| Mittel_GAOS0353-01301 | -82,0 | 1775,4 |
| Mittel_GAOS0353-01502 | -18,3 | 1735,3 |

Mit zunehmender SSII-Aktivität nimmt die Klebrigkeit der gekochten Reiskörner ab (Tab. 4a). Die Reduktion der Klebrigkeit ist in Linie GAOS0353-02301 auf ca. die Hälfte, in Linie GAOS0353-01301 um etwa den Faktor 3 und in Linie GAOS0353-01502 um ca. den Faktor 13 verringert.

**Tabelle 4b: Textur von frisch gekochten bzw. wiedererwärmten Reiskörnern**

| Aufarbeitung der gemessenen Körner | Probe | Klebrigkeit (g) | Klebrigkeit (%) |
|---|---|---|---|
| Frisch gekocht | Wildtyp M202 | - 235,8 | 100,0 |
| | GAOS0353-01502 | - 121,4 | 51,5 |
| Wiedererwärmung im Ofen, 80°C / 5 min | Wildtyp M202 | - 230,5 | 100,0 |
| | GAOS0353-01502 | - 95,0 | 41,2 |
| Wiedererwärmung in Mikrowelle, 600W/ 3 min | Wildtyp M202 | - 182,2 | 100,0 |
| | GAOS0353-01502 | - 85,3 | 46,8 |

Zusammenfassung der Messdaten zur Bestimmung der Klebrigkeit gekochter Reiskörner nach dem Kochen sowie Wiedererwärmung im Ofen bzw. in der Mikrowelle (nach Lagerung für 22h bei 4°C). Die Klebrigkeit der Linie GAOS0353-01502 liegt bei jeder Aufarbeitungs-Variante erheblich unter der des Wildtyps.

### Beispiel 6: Bestimmung der Korndimensionen von ungekochten und gekochten Reiskörnern

Die Korndimensionen von ungekochten und gekochten Reiskörnern genetisch modifizierter Linien sowie des entsprechenden Wildtyps wurden mittels der SigmScan Software bestimmt. Die Ergebnisse und die daraus abgeleiteten Parameter sind in den Tabellen 5a und b dargestellt.

**Tabelle 5a: Zusammenfassung der Messdaten zur Bestimmung der Korndimensionen von ungekochten und gekochten Reiskörnern.**

| | Wildtyp | GAOS035302301 | GAOS035301301 | GAOS035301502 |
|---|---|---|---|---|
| Lu (in mm) | 5,36 | 4,81 | 4,94 | 4,87 |
| Bu (in mm) | 2,60 | 2,58 | 2,65 | 2,50 |
| Lc (in mm) | 7,59 | 7,50 | 8,18 | 7,87 |
| Bc (in mm) | 3,74 | 3,61 | 3,23 | 3,26 |
| ER | 1,41 | 1,56 | 1,65 | 1,62 |
| Lc/Bc | 2,05 | 2,10 | 2,56 | 2,43 |
| CDC | 2,20 | 2,95 | 4,92 | 4,39 |

| | | | | |
|---|---|---|---|---|
| Angegeben sind die Mittelwerte von 30 unabhängigen Messungen (L=Kornlänge; B=Kornbreite; u=ungekocht; c=gekocht; ER = Elongationsrate (Lc/Lu): CDC=Coeffizient of dimensional changes (Lc/Lu)/(Bc/Bu)). | | | | |

**Tabelle 5b: Relative Veränderungen der Korndimensionen im Vergleich zum Wildtyp**

| | Wildtyp | GAOS035302301 | GAOS035301301 | GAOS035301502 |
|---|---|---|---|---|
| Lu | 0 | -10,3 | -7,8 | -9,1 |
| Bu | 0 | -0,8 | 1,9 | -3,8 |
| Lc | 0 | -1,2 | 7,8 | 3,7 |
| Bc | 0 | -3,5 | -13,6 | -12,8 |
| ER | 0 | 10,6 | 17,0 | 14,9 |
| Lc/Bc | 0 | 2,4 | 24,9 | 18,5 |
| CDC | 0 | 34,1 | 123,6 | 99,5 |

| | | | | |
|---|---|---|---|---|
| Alle Angaben in Prozent: % Veränderung = (Probe - Wildtyp)/Wildtyp*100). | | | | |

Im Hinblick auf die Korndimensionen von gekochten Reiskörnern führt die erhöhte SSII- Aktivität in Reis zu einer signifikant verstärkten Ausdehnung der Körner während des Kochens in Richtung der Längsachse. Dies wird durch die erhöhte Elongationsrate (ER) und das erhöhte Längenbreiten-Verhältnis (Lc/Bc) deutlich. Auch hier zeigt die Linie mit einer zweifach erhöhten SSII-Aktivität (GAOS0353-02301) eine geringere Änderung auf die oben beschriebenen Parameter, während die beiden Linien mit sechsfach (GAOS0353-01301) bzw. zehnfach erhöhter SSII-Aktivität (GAOS0353-01502) deutlich stärkere Ausprägungen aufweisen.

### Beispiel 7: Analyse der physiko-chemischen Eigenschaften von Reismehl mittels Rapid Visco Analyzer (RVA)

Reismehle verschiedener genetisch modifizierten Linien sowie des entsprechenden Wildtyps wurden gemäß der Methode "Analyse von Reismehl mittels RVA" hinsichtlich ihrer physiko-chemischen Eigenschaften analysiert.
Die Viskosität der Reismehl/Wasser-Suspension wurde über den Verlauf eines definierten Temperatur- und Scherprogramms aufgezeichnet. Die Graphen bzw. Messwerte der Analyse der verschiedenen Linien sind in Tabelle 6 a+b dargestellt.

**Tabelle 6a: Zusammenfassung der Messdaten zur Bestimmung des Viskositätsverhaltens von Reismehlen hergestellt aus Reiskörnern von genetisch modifizierten Linien mit unterschiedlicher SSII-Aktivität**

| Probe: | Wildtyp | 353-02301 | 353-01301 | 353-01502 |
|---|---|---|---|---|
| Peak Viskosität (cP) | 4767 | 4626 | 4322 | 3787 |
| Trough (cP) | 2122 | 1755 | 1647 | 1515 |
| Breakdown (cP) | 2645 | 2871 | 2675 | 2272 |
| End Viskosität (cP) | 2934 | 2338 | 2249 | 2065 |
| Setback (cP) | 812 | 583 | 602 | 550 |
| Setback rice (cP) | -1833 | -2288 | -2073 | -1722 |
| Peak Time (min) | 5,36 | 5,02 | 5,04 | 4,56 |
| Pasting Temperatur (°C) | 72,8 | 82,3 | 81,7 | 83,9 |
| Pasting Time (min) | 2,56 | 3,36 | 3,4 | 3,48 |
| Peak Time - Pasting Time (sec) | 144,0 | 73,2 | 76,8 | 64,8 |

**Tabelle 6b: Relative Veränderungen der RVA Parameter im Vergleich zum Wildtyp**

| Probe: | Wildtyp | 353-02301 | 353-01301 | 353-01502 |
|---|---|---|---|---|
| Peak Viskosität (cP) | 0,0 | - 3,0 | - 9,3 | - 20,6 |
| Trough (cP) | 0,0 | - 17,3 | - 22,4 | - 28,6 |
| Breakdown (cP) | 0,0 | 8,5 | 1,1 | - 14,1 |
| End Viskosität (cP) | 0,0 | - 20,3 | - 23,3 | - 29,6 |
| Setback (cP) | 0,0 | - 28,2 | - 25,9 | - 32,3 |
| Setback rice (cP) | 0,0 | 24,8 | 13,1 | - 6,1 |
| Peak Time (min) | 0,0 | - 9,5 | - 9,5 | - 12,0 |
| Pasting Temperatur (°C) | 0,0 | 13,0 | 12,2 | 15,2 |
| Pasting Time (min) | 0,0 | 32,8 | 31,3 | 35,9 |
| Peak Time - Pasting Time (min) | 0,0 | - 49,2 | - 46,7 | - 55,0 |

(alle Angaben in Prozent) %Veränderung = (Probe - Wildtyp)/Wildtyp*100
Der Verlauf der Viskosität von Mehlen des Wildtyps und der genetisch modifizierten Linien unterscheidet sich in mehreren Parametern. Die genetisch modifizierten Proben fangen zu einem deutlich späterer Zeitpunkt an zu verkleistern, was an der erhöhten "Pasting temperature" (Verkleisterungstemperatur) erkennbar ist. Die darauf folgende Entwicklung der Viskosität bis hin zur Peak-Viskosität erfolgt sehr schnell, wie an der geringeren "Peak time" der genetisch modifizierten Proben deutlich wird. Alle weiteren Viskositätsparameter (Peak, Trough, Final) fallen für die genetisch modifizierten Proben geringer aus als für den Wildtyp. Hierbei ist festzuhalten, dass das Ausmaß der Veränderungen gegenüber dem Wildtyp mit der Höhe der SSII-Aktivität korreliert. Die Linie mit der höchsten SSII-Aktivität (GAOS0353-1502) zeigt die geringste Peak-, Trough und End-Viskosität und weist mit mehr als 10°C den größten Unterschied in der Verkleisterungstemperatur auf.
Ebenfalls sehr auffällig ist die sehr schnelle Viskositätsentwicklung der genetisch modifizierten Proben, die sich in einem sehr kurzen Abstand zwischen des Zeitpunktes des Verkleisterungsbeginn und des Zeitpunktes der Peak-Viskosität zeigt. Auch für diesen Parameter ist eine deutliche Abhängigkeit des Effektes von dem Grad der Erhöhung der SSII-Aktivität zu erkennen.

## Patentansprüche

1. Reisstärke **dadurch gekennzeichnet, dass** sie eine DSC T-onset Temperatur zwischen 70°C und 80°C aufweist.

2. Reisstärke nach Anspruch 1, wobei die DSC T-onset Temperatur zwischen 72°C und 79°C liegt.

3. Derivatisierte Reisstärke, enthaltend Reisstärke nach Anspruch 1 oder 2.

4. Zusammensetzung, enthaltend Reisstärke nach einem der Ansprüche 1 bis 3.

5. Reismehl, **dadurch gekennzeichnet, dass** es Reisstärke nach Anspruch 1 oder 2 enthält.

6. Zusammensetzung, enthaltend Reismehl nach Anspruch 5.

7. Reiskorn, enthaltend Reisstärke nach Anspruch 1 oder 2.

8. Zusammensetzung, enthaltend mindestens ein Reiskorn nach Anspruch 7.

9. Reispflanze, tragend mindestens ein Reiskorn nach Anspruch 7 und/oder enthaltend Reisstärke nach Anspruch 1 oder 2.


SEQUENCE LISTING

<110> Bayer CropScience GmbH

<120> Reisstärken mit verbesserten Qualitätseigenschaften

<130> BCS 05-5011-EP

<160> 5

<170> PatentIn version 3.1

<210> 1
<211> 3006
<212> DNA
<213> Triticum aestivum

<220>
<221> CDS
<222> (227)..(2623)
<223>

<220>
<221> Region1
<222> (1190)..(1279)
<223>

<220>
<221> Region2
<222> (1493)..(1612)
<223>

<220>
<221> Region3
<222> (2147)..(2350)

<223>

<300>

<302>  Nucleic acid molecules encoding enzymes from wheat which are involved in
starch synthesis

<308>  Derwent/AAV01528

<309>  1998-05-21

<310>  wo 97 45545

<311>  1997-05-28

<312>  1997-12-04

<313>  (1)..(2825)

<400>  1
ctccaccgcg gtggcggccg ctctagaact agtggatccc ccgggctgca ggaattcggc      60

acgagcttcg gcctgacccc gttcgtttac ccccacacag agcacactcc agtccagtcc     120

agcccactgc caccgcgcta ctctccactc ccactgccac cacctccgcc tgcgccgcgc     180

tctgggcgga ccaacccgcg aaccgtacca tctcccgccc cgatcc atg tcg tcg       235
                                                   Met Ser Ser
                                                   1

gcg gtc gcg tcc gcc gca tcc ttc ctc gcg ctc gcg tca gcc tcc ccc       283
Ala Val Ala Ser Ala Ala Ser Phe Leu Ala Leu Ala Ser Ala Ser Pro
    5                   10                  15

ggg aga tca cgc agg cgg gcg agg gtg agc gcg cag cca ccc cac gcc       331
Gly Arg Ser Arg Arg Arg Ala Arg Val Ser Ala Gln Pro Pro His Ala
20                  25                  30                  35

ggg gcc ggc agg ttg cac tgg ccg ccg tgg ccg ccg cag cgc acg gct       379
Gly Ala Gly Arg Leu His Trp Pro Pro Trp Pro Pro Gln Arg Thr Ala
                40                  45                  50

cgc gac gga gct gtg gcg gcg ctc gcc gcc ggg aag aag gac gcg ggg       427
Arg Asp Gly Ala Val Ala Ala Leu Ala Ala Gly Lys Lys Asp Ala Gly
            55                  60                  65

atc gac gac gcc gcc gcg tcc gtg agg cag ccc cgc gca ctc cgc ggt       475
Ile Asp Asp Ala Ala Ala Ser Val Arg Gln Pro Arg Ala Leu Arg Gly
        70                  75                  80

ggc gcc gcc acc aag gtc gcg gag cga agg gat ccc gtc aag acg ctc       523
Gly Ala Ala Thr Lys Val Ala Glu Arg Arg Asp Pro Val Lys Thr Leu
    85                  90                  95

gac cgc gac gcc gcg gaa ggc ggc ggg ccg tcc ccg ccg gca gcg agg       571
Asp Arg Asp Ala Ala Glu Gly Gly Gly Pro Ser Pro Pro Ala Ala Arg
100                 105                 110                 115

cag gac gcc gcc cgt ccg ccg agt atg aac ggc atg ccg gtg aac ggc       619
Gln Asp Ala Ala Arg Pro Pro Ser Met Asn Gly Met Pro Val Asn Gly
                120                 125                 130

gag aac aaa tct acc ggc ggc ggc ggc gcg act aaa gac agc ggg ctg       667

Glu Asn Lys Ser Thr Gly Gly Gly Gly Ala Thr Lys Asp Ser Gly Leu
            135                 140                 145

ccc acg ccc gca cgc gcg ccc cat ccg tcg acc cag aac aga gca ccg 715
Pro Thr Pro Ala Arg Ala Pro His Pro Ser Thr Gln Asn Arg Ala Pro
        150                 155                 160

gtg aac ggt gaa aac aaa gct aac gtc gcc tcg ccg ccg acg agc ata 763
Val Asn Gly Glu Asn Lys Ala Asn Val Ala Ser Pro Pro Thr Ser Ile
    165                 170                 175

gcc gag gcc gcg gct tcg gat tcc gca gct acc att tcc atc agc gac 811
Ala Glu Ala Ala Ala Ser Asp Ser Ala Ala Thr Ile Ser Ile Ser Asp
180                 185                 190                 195

aag gcg ccg gag tcc gtt gtc cca gct gag aag acg ccg ccg tcg tcc 859
Lys Ala Pro Glu Ser Val Val Pro Ala Glu Lys Thr Pro Pro Ser Ser
                200                 205                 210

ggc tca aat ttc gag tcc tcg gcc tct gct ccc ggg tct gac act gtc 907
Gly Ser Asn Phe Glu Ser Ser Ala Ser Ala Pro Gly Ser Asp Thr Val
            215                 220                 225

agc gac gtg gaa caa gaa ctg aag aag ggt gcg gtc gtt gtc gaa gaa 955
Ser Asp Val Glu Gln Glu Leu Lys Lys Gly Ala Val Val Val Glu Glu
        230                 235                 240

gct cca aag cca aag gct ctt tcg ccg cct gca gcc ccc gct gta caa 1003
Ala Pro Lys Pro Lys Ala Leu Ser Pro Pro Ala Ala Pro Ala Val Gln
    245                 250                 255

gaa gac ctt tgg gat ttc aag aaa tac att ggt ttc gag gag ccc gtg 1051
Glu Asp Leu Trp Asp Phe Lys Lys Tyr Ile Gly Phe Glu Glu Pro Val
260                 265                 270                 275

gag gcc aag gat gat ggc cgg gct gtc gca gat gat gcg ggc tcc ttt 1099
Glu Ala Lys Asp Asp Gly Arg Ala Val Ala Asp Asp Ala Gly Ser Phe
                280                 285                 290

gaa cac cac cag aat cac gac tcc gga cct ttg gca ggg gag aat gtc 1147
Glu His His Gln Asn His Asp Ser Gly Pro Leu Ala Gly Glu Asn Val
            295                 300                 305

atg aac gtg gtc gtc gtg gct gct gag tgt tct ccc tgg tgc aaa aca 1195
Met Asn Val Val Val Val Ala Ala Glu Cys Ser Pro Trp Cys Lys Thr
        310                 315                 320

ggt ggt ctg gga gat gtt gcg ggt gct ctg ccc aag gct ttg gca aag 1243
Gly Gly Leu Gly Asp Val Ala Gly Ala Leu Pro Lys Ala Leu Ala Lys
    325                 330                 335

aga gga cat cgt gtt atg gtt gtg gta cca agg tat ggg gac tat gaa 1291
Arg Gly His Arg Val Met Val Val Val Pro Arg Tyr Gly Asp Tyr Glu
340                 345                 350                 355

gaa gcc tac gat gtc gga gtc cga aaa tac tac aag gct gct gga cag 1339
Glu Ala Tyr Asp Val Gly Val Arg Lys Tyr Tyr Lys Ala Ala Gly Gln
                360                 365                 370

gat atg gaa gtg aat tat ttc cat gct tat atc gat gga gtt gat ttt 1387
Asp Met Glu Val Asn Tyr Phe His Ala Tyr Ile Asp Gly Val Asp Phe
            375                 380                 385

gtg ttc att gac gct cct ctc ttc cga cac cgt cag gaa gac att tat 1435
Val Phe Ile Asp Ala Pro Leu Phe Arg His Arg Gln Glu Asp Ile Tyr
        390                 395                 400

ggg ggc agc aga cag gaa att atg aag cgc atg att ttg ttc tgc aag 1483

Gly Gly Ser Arg Gln Glu Ile Met Lys Arg Met Ile Leu Phe Cys Lys
    405                 410                 415

gcc gct gtt gag gtt cca tgg cac gtt cca tgc ggc ggt gtc cct tat      1531
Ala Ala Val Glu Val Pro Trp His Val Pro Cys Gly Gly Val Pro Tyr
420                 425                 430                 435

ggg gat gga aat ctg gtg ttt att gca aat gat tgg cac acg gca ctc      1579
Gly Asp Gly Asn Leu Val Phe Ile Ala Asn Asp Trp His Thr Ala Leu
                440                 445                 450

ctg cct gtc tat ctg aaa gca tat tac agg gac cat ggt ttg atg cag      1627
Leu Pro Val Tyr Leu Lys Ala Tyr Tyr Arg Asp His Gly Leu Met Gln
            455                 460                 465

tac act cgg tcc att atg gtg ata cat aac atc gct cac cag ggc cgt      1675
Tyr Thr Arg Ser Ile Met Val Ile His Asn Ile Ala His Gln Gly Arg
        470                 475                 480

ggc cct gta gat gaa ttc ccg ttc acc gag ttg cct gag cac tac ctg      1723
Gly Pro Val Asp Glu Phe Pro Phe Thr Glu Leu Pro Glu His Tyr Leu
    485                 490                 495

gaa cac ttc aga ctg tac gac ccc gtg ggt ggt gaa cac gcc aac tac      1771
Glu His Phe Arg Leu Tyr Asp Pro Val Gly Gly Glu His Ala Asn Tyr
500                 505                 510                 515

ttc gcc gcc ggc ctg aag atg gcg gac cag gtt gtc gtg gtg agc ccc      1819
Phe Ala Ala Gly Leu Lys Met Ala Asp Gln Val Val Val Val Ser Pro
                520                 525                 530

ggg tac ctg tgg gag ctg aag acg gtg gag ggc ggc tgg ggg ctt cac      1867
Gly Tyr Leu Trp Glu Leu Lys Thr Val Glu Gly Gly Trp Gly Leu His
            535                 540                 545

gac atc ata cgg cag aac gac tgg aag acc cgc ggc atc gtc aac ggc      1915
Asp Ile Ile Arg Gln Asn Asp Trp Lys Thr Arg Gly Ile Val Asn Gly
        550                 555                 560

atc gac aac atg gag tgg aac ccc gag gtg gac gcc cac ctc aag tcg      1963
Ile Asp Asn Met Glu Trp Asn Pro Glu Val Asp Ala His Leu Lys Ser
    565                 570                 575

gac ggc tac acc aac ttc tcc ctg agg acg ctg gac tcc ggc aag cgg      2011
Asp Gly Tyr Thr Asn Phe Ser Leu Arg Thr Leu Asp Ser Gly Lys Arg
580                 585                 590                 595

cag tgc aag gag gcc ctg cag cgc gag ctg ggc ctg cag gtc cgc gcc      2059
Gln Cys Lys Glu Ala Leu Gln Arg Glu Leu Gly Leu Gln Val Arg Ala
                600                 605                 610

gac gtg ccg ctg ctc ggc ttc atc ggc cgc ctg gac ggg cag aag ggc      2107
Asp Val Pro Leu Leu Gly Phe Ile Gly Arg Leu Asp Gly Gln Lys Gly
            615                 620                 625

gtg gag atc atc gcg gac gcc atg ccc tgg atc gtg agc cag gac gtg      2155
Val Glu Ile Ile Ala Asp Ala Met Pro Trp Ile Val Ser Gln Asp Val
        630                 635                 640

cag ctg gtg atg ctg ggc acc ggg cgc cac gac ctg gag agc atg ctg      2203
Gln Leu Val Met Leu Gly Thr Gly Arg His Asp Leu Glu Ser Met Leu
    645                 650                 655

cag cac ttc gag cgg gag cac cac gac aag gtg cgc ggg tgg gtg ggg      2251
Gln His Phe Glu Arg Glu His His Asp Lys Val Arg Gly Trp Val Gly
660                 665                 670                 675

ttc tcc gtg cgc ctg gcg cac cgg atc acg gcg ggg gcg gac gcg ctc      2299

Phe Ser Val Arg Leu Ala His Arg Ile Thr Ala Gly Ala Asp Ala Leu
                680                 685                 690

ctc atg ccc tcc cgg ttc gag ccg tgc ggg ctg aac cag ctc tac gcc      2347
Leu Met Pro Ser Arg Phe Glu Pro Cys Gly Leu Asn Gln Leu Tyr Ala
            695                 700                 705

atg gcc tac ggc acc gtc ccc gtc gtg cac gcc gtc ggc ggc ctc agg      2395
Met Ala Tyr Gly Thr Val Pro Val Val His Ala Val Gly Gly Leu Arg
        710                 715                 720

gac acc gtg ccg ccg ttc gac ccc ttc aac cac tcc ggg ctc ggg tgg      2443
Asp Thr Val Pro Pro Phe Asp Pro Phe Asn His Ser Gly Leu Gly Trp
    725                 730                 735

acg ttc gac cgc gcc gag gcg cac aag ctg atc gag gcg ctc ggg cac      2491
Thr Phe Asp Arg Ala Glu Ala His Lys Leu Ile Glu Ala Leu Gly His
740                 745                 750                 755

tgc ctc cgc acc tac cga gac ttc aag gag agc tgg agg gcc ctc cag      2539
Cys Leu Arg Thr Tyr Arg Asp Phe Lys Glu Ser Trp Arg Ala Leu Gln
                760                 765                 770

gag cgc ggc atg tcg cag gac ttc agc tgg gag cac gcc gcc aag ctc      2587
Glu Arg Gly Met Ser Gln Asp Phe Ser Trp Glu His Ala Ala Lys Leu
            775                 780                 785

tac gag gac gtc ctc gtc aag gcc aag tac cag tgg tgaacgctag           2633
Tyr Glu Asp Val Leu Val Lys Ala Lys Tyr Gln Trp
        790                 795

ctgctagccg ctccagcccc gcatgcgtgc atgacaggat ggaactgcat tgcgcacgca    2693

ggaaagtgcc atggagcgcc ggcatccgcg aagtacagtg acatgaggtg tgtgtggttg    2753

agacgctgat tccaatccgg cccgtagcag agtagagcgg aggtatatgg gaatcttaac    2813

ttggtattgt aatttgttat gttgtgtgca ttattacaat gttgttactt attcttgtta    2873

agtcggaggc caagggcgaa agctagctca catgtctgat ggatgcacgt gccatggttg    2933

gtttggtagc gcagtgcaaa cggcaagaat gggaagtgaa ttcctccctg cttgaaaaaa    2993

aaaaaaaaaa aaa                                                       3006

<210>  2

<211>  799

<212>  PRT

<213>  Triticum aestivum


<400>  2

Met Ser Ser Ala Val Ala Ser Ala Ala Ser Phe Leu Ala Leu Ala Ser
1               5                   10                  15


Ala Ser Pro Gly Arg Ser Arg Arg Arg Ala Arg Val Ser Ala Gln Pro
            20                  25                  30


Pro His Ala Gly Ala Gly Arg Leu His Trp Pro Pro Trp Pro Pro Gln
        35                  40                  45

Arg Thr Ala Arg Asp Gly Ala Val Ala Ala Leu Ala Ala Gly Lys Lys
    50                  55                  60

Asp Ala Gly Ile Asp Asp Ala Ala Ala Ser Val Arg Gln Pro Arg Ala
65                  70                  75                  80

Leu Arg Gly Gly Ala Ala Thr Lys Val Ala Glu Arg Arg Asp Pro Val
                85                  90                  95

Lys Thr Leu Asp Arg Asp Ala Ala Glu Gly Gly Gly Pro Ser Pro Pro
            100                 105                 110

Ala Ala Arg Gln Asp Ala Ala Arg Pro Pro Ser Met Asn Gly Met Pro
        115                 120                 125

Val Asn Gly Glu Asn Lys Ser Thr Gly Gly Gly Gly Ala Thr Lys Asp
    130                 135                 140

Ser Gly Leu Pro Thr Pro Ala Arg Ala Pro His Pro Ser Thr Gln Asn
145                 150                 155                 160

Arg Ala Pro Val Asn Gly Glu Asn Lys Ala Asn Val Ala Ser Pro Pro
                165                 170                 175

Thr Ser Ile Ala Glu Ala Ala Ala Ser Asp Ser Ala Ala Thr Ile Ser
            180                 185                 190

Ile Ser Asp Lys Ala Pro Glu Ser Val Val Pro Ala Glu Lys Thr Pro
        195                 200                 205

Pro Ser Ser Gly Ser Asn Phe Glu Ser Ser Ala Ser Ala Pro Gly Ser
    210                 215                 220

Asp Thr Val Ser Asp Val Glu Gln Glu Leu Lys Lys Gly Ala Val Val
225                 230                 235                 240

Val Glu Glu Ala Pro Lys Pro Lys Ala Leu Ser Pro Pro Ala Ala Pro
                245                 250                 255

Ala Val Gln Glu Asp Leu Trp Asp Phe Lys Lys Tyr Ile Gly Phe Glu
            260                 265                 270

Glu Pro Val Glu Ala Lys Asp Asp Gly Arg Ala Val Ala Asp Asp Ala
        275                 280                 285

Gly Ser Phe Glu His His Gln Asn His Asp Ser Gly Pro Leu Ala Gly
    290                 295                 300

Glu Asn Val Met Asn Val Val Val Val Ala Ala Glu Cys Ser Pro Trp
305                 310                 315                 320

Cys Lys Thr Gly Gly Leu Gly Asp Val Ala Gly Ala Leu Pro Lys Ala
                325                 330                 335

Leu Ala Lys Arg Gly His Arg Val Met Val Val Val Pro Arg Tyr Gly
            340                 345                 350

Asp Tyr Glu Glu Ala Tyr Asp Val Gly Val Arg Lys Tyr Tyr Lys Ala
        355                 360                 365

Ala Gly Gln Asp Met Glu Val Asn Tyr Phe His Ala Tyr Ile Asp Gly
    370                 375                 380

Val Asp Phe Val Phe Ile Asp Ala Pro Leu Phe Arg His Arg Gln Glu
385                 390                 395                 400

Asp Ile Tyr Gly Gly Ser Arg Gln Glu Ile Met Lys Arg Met Ile Leu
                405                 410                 415

Phe Cys Lys Ala Ala Val Glu Val Pro Trp His Val Pro Cys Gly Gly
            420                 425                 430

Val Pro Tyr Gly Asp Gly Asn Leu Val Phe Ile Ala Asn Asp Trp His
        435                 440                 445

Thr Ala Leu Leu Pro Val Tyr Leu Lys Ala Tyr Tyr Arg Asp His Gly
    450                 455                 460

Leu Met Gln Tyr Thr Arg Ser Ile Met Val Ile His Asn Ile Ala His
465                 470                 475                 480

Gln Gly Arg Gly Pro Val Asp Glu Phe Pro Phe Thr Glu Leu Pro Glu
                485                 490                 495

His Tyr Leu Glu His Phe Arg Leu Tyr Asp Pro Val Gly Gly Glu His
            500                 505                 510

Ala Asn Tyr Phe Ala Ala Gly Leu Lys Met Ala Asp Gln Val Val Val
        515                 520                 525

Val Ser Pro Gly Tyr Leu Trp Glu Leu Lys Thr Val Glu Gly Gly Trp
    530                 535                 540

Gly Leu His Asp Ile Ile Arg Gln Asn Asp Trp Lys Thr Arg Gly Ile
545                 550                 555                 560

Val Asn Gly Ile Asp Asn Met Glu Trp Asn Pro Glu Val Asp Ala His
                565                 570                 575

Leu Lys Ser Asp Gly Tyr Thr Asn Phe Ser Leu Arg Thr Leu Asp Ser
            580                 585                 590

Gly Lys Arg Gln Cys Lys Glu Ala Leu Gln Arg Glu Leu Gly Leu Gln
        595                 600                 605

Val Arg Ala Asp Val Pro Leu Leu Gly Phe Ile Gly Arg Leu Asp Gly
    610                 615                 620

Gln Lys Gly Val Glu Ile Ile Ala Asp Ala Met Pro Trp Ile Val Ser
625                 630                 635                 640

Gln Asp Val Gln Leu Val Met Leu Gly Thr Gly Arg His Asp Leu Glu
                645                 650                 655

Ser Met Leu Gln His Phe Glu Arg Glu His His Asp Lys Val Arg Gly
            660                 665                 670

Trp Val Gly Phe Ser Val Arg Leu Ala His Arg Ile Thr Ala Gly Ala
        675                 680                 685

Asp Ala Leu Leu Met Pro Ser Arg Phe Glu Pro Cys Gly Leu Asn Gln
    690                 695                 700

Leu Tyr Ala Met Ala Tyr Gly Thr Val Pro Val Val His Ala Val Gly
705                 710                 715                 720

Gly Leu Arg Asp Thr Val Pro Pro Phe Asp Pro Phe Asn His Ser Gly
                725                 730                 735

Leu Gly Trp Thr Phe Asp Arg Ala Glu Ala His Lys Leu Ile Glu Ala
            740                 745                 750

Leu Gly His Cys Leu Arg Thr Tyr Arg Asp Phe Lys Glu Ser Trp Arg
        755                 760                 765

Ala Leu Gln Glu Arg Gly Met Ser Gln Asp Phe Ser Trp Glu His Ala
    770                 775                 780

Ala Lys Leu Tyr Glu Asp Val Leu Val Lys Ala Lys Tyr Gln Trp
785                 790                 795

<210>  3

<211>  30

<212>  PRT

<213>  Triticum aestivum


<400>  3

Lys Thr Gly Gly Leu Gly Asp Val Ala Gly Ala Leu Pro Lys Ala Leu
1               5                   10                  15

Ala Lys Arg Gly His Arg Val Met Val Val Val Pro Arg Tyr
            20                  25                  30

<210>  4

<211>  40

<212>  PRT

<213>  Triticum aestivum

<400>  4

Glu Val Pro Trp His Val Pro Cys Gly Gly Val Pro Tyr Gly Asp Gly
1               5                   10                  15

Asn Leu Val Phe Ile Ala Asn Asp Trp His Thr Ala Leu Leu Pro Val
            20                  25                  30

Tyr Leu Lys Ala Tyr Tyr Arg Asp
        35                  40

<210>  5

<211>  68

<212>  PRT

<213>  Triticum aestivum

<400>  5

Gln Asp Val Gln Leu Val Met Leu Gly Thr Gly Arg His Asp Leu Glu
1               5                   10                  15

Ser Met Leu Gln His Phe Glu Arg Glu His His Asp Lys Val Arg Gly
            20                  25                  30

Trp Val Gly Phe Ser Val Arg Leu Ala His Arg Ile Thr Ala Gly Ala
        35                  40                  45

Asp Ala Leu Leu Met Pro Ser Arg Phe Glu Pro Cys Gly Leu Asn Gln
    50                  55                  60

Leu Tyr Ala Met
65